(19) Europäisches Patentamt
European Patent Office
Office européen des brevets

(11) Veröffentlichungsnummer: **0 261 539 B1**

(12) # EUROPÄISCHE PATENTSCHRIFT

(45) Veröffentlichungstag der Patentschrift: **06.03.91**

(51) Int. Cl.[5]: **C07D 215/26**, C07D 215/20, C07D 215/227, C07D 215/14, C07D 213/74, C07D 213/38, A61K 31/47, A61K 31/44

(21) Anmeldenummer: **87113393.0**

(22) Anmeldetag: **14.09.87**

Die Akte enthält technische Angaben, die nach dem Eingang der Anmeldung eingereicht wurden und die nicht in dieser Patentschrift enthalten sind.

(54) **Substituierte Phenylsulfonamide.**

(30) Priorität: **24.09.86 DE 3632329**

(43) Veröffentlichungstag der Anmeldung:
**30.03.88 Patentblatt 88/13**

(45) Bekanntmachung des Hinweises auf die Patenterteilung:
**06.03.91 Patentblatt 91/10**

(84) Benannte Vertragsstaaten:
**AT BE CH DE ES FR GB IT LI NL SE**

(56) Entgegenhaltungen:

| | |
|---|---|
| **EP-A- 0 114 734** | **EP-A- 0 232 954** |
| **GB-A- 2 181 135** | **GB-A- 4 661 499** |
| **JP-A- 6 101 548** | **US-A- 4 563 526** |

(73) Patentinhaber: **BAYER AG**

**W-5090 Leverkusen 1 Bayerwerk(DE)**

(72) Erfinder: **Mohrs, Klaus, Dr.**
**Claudiusweg 9**
**W-5600 Wuppertal 1(DE)**
Erfinder: **Perzborn, Elisabeth, Dr.**
**Am Tescher Busch 13**
**W-5600 Wuppertal 11(DE)**
Erfinder: **Seuter, Friedel, Dr.**
**Moospfad 16**
**W-5600 Wuppertal 1(DE)**
Erfinder: **Fruchtmann, Romanis, Dipl.-Biologin**
**Konrad-Adenauer-Ufer 71**
**W-5000 Köln 1(DE)**
Erfinder: **Kohlsdorfer, Christian, Dr.**
**Franz-Stryck-Strasse 16**
**W-5042 Erftstadt(DE)**

EP 0 261 539 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft substituierte Phenylsulfonamide, Verfahren zu ihrer Herstellung und ihre Verwendung in Arzneimitteln.

Aus US-Patentschrift 4 581 457 ist bekannt, das Phenylsulfonamide mit einer Benzimidazolylmethoxygruppe oder mit einer Benzothiazolylmethoxygruppe im Aromaten antiinflammatorische Wirkung haben.

O-Pyridyl-benzylsulfonamide werden in JP 61/010548 mit einer antiinflammatorischen und antithrombotischen Wirkung und in CA 101, 110849 v mit einer Pflanzenschutzwirkung beschrieben.

In der EP 232 954 werden Chinolinyl-methoxy-substituierte Phenylsulfonamide beschrieben, in denen der Sulfonamid-Rest in 3-Position zum Chinolinyl-methoxy-Rest gebunden ist.

Die vorliegende Erfindung betrifft neue substituierte Phenyl-sulfonamide der allgemeinen Formel (I)

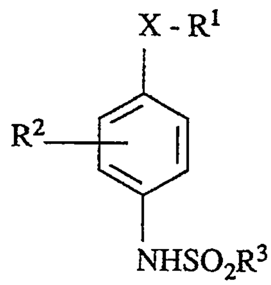

(I)

worin

- $R^1$- für einen Chinolyl- oder Isochinolylrest steht, der substituiert sein kann durch Fluor, Chlor, Brom, $C_1$-$C_6$-Alkyl, Cyclopropyl, Cyclopentyl,Cyclohexyl, $C_1$-$C_6$-Alkoxy, Cyano, Trifluoromethyl Trifluormethoxy, Alkoxycarbonyl mit 1 bis 6 C-Atomen im Alkylteil, $C_1$-$C_6$-Alkylsulfonyl,
- $R^2$- für Wasserstoff, Cyano, Nitro, Fluor, Chlor, Brom,$C_1$-$C_6$-Alkyl,$C_1$-$C_6$-Alkoxy, Trifluoromethyl Trifluormethoxy oder Alkoxycarbonyl mit 1 bis 6 C-Atomen im Alkylteil steht,
- $R^3$- für Phenyl steht, das bis zu 2-fach gleich oder verschieden substituiert sein kann durch Fluor, Chlor, Brom, Trifluormethyl, Trifluormethoxy, $C_1$-$C_6$-Alkyl, $C_1$-$C_6$-Alkylthio, $C_1$-$C_6$-Alkylsulfonyl, Cyano, Alkoxycarbonyl mit 1 bis 6 C-Atomen im Alkylteil oder
  - für Pentafluorphenyl oder
  - für geradkettiges,, verzweigtes oder cyclisches Alkyl mit bis zu 8 Kohlenstoffatomen steht,, das substituiert sein kann durch Fluor, Chlor, Brom, Phenyl, Phenoxy, Cyano, $C_1$-$C_6$-Alkoxy oder Trifluormethyl

und

X - für eine Gruppierung -O-, -A-B- oder -B-A- steht,

wobei

A - -O -, 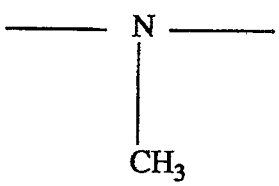 oder 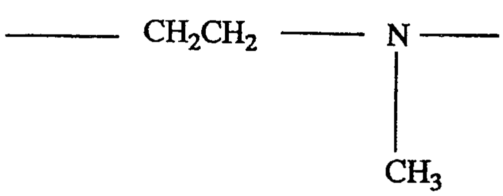 bedeutet

und

B - $-CH_2-$ oder 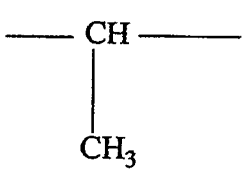 bedeutet,

und deren Salze.

Die erfindungsgemäßen substituierten Phenylsulfonamide können auch in Form ihrer Salze vorliegen. Im allgemeinen seien hier Salze mit organischen oder anorganischen Säuren genannt.

Im Rahmen der vorliegenden Erfindung werden physiologisch unbedenkliche Salze bevorzugt. Physiologisch unbedenkliche Salze der substituierten Phenylsulfonamide können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Die erfindungsgemäßen Stoffe zeigen überraschenderweise eine gute antiinflammatorische und thrombozytenaggregationshemmende Wirkung und können zur therapeutischen Behandlung von Menschen und Tieren verwendet werden. Bevorzugt werden Verbindungen der allgemeinen Formel (I) in welcher

$R^1$- für einen Chinolyl- oder Isochinolylrest steht, der substituiert sein kann durch Fluor, Chlor, Alkyl mit bis zu 4 Kohlenstoffatomen, Alkoxy mit bis zu 4 Kohlenstoffatomen oder durch Trifluormethyl,

$R^2$- für Wasserstoff, Cyano, Fluor, Chlor, Methyl, Ethyl, Fropyl, Isopropyl, Methoxy, Ethoxy, Trifluormethyl, Methoxycarbonyl, Ethoxycarbonyl oder Propoxycarbonyl steht,

$R^3$ - für Phenyl steht, das substituiert sein kann durch Fluor, Chlor, Trifluormethyl, Trilfuormethoxy, Alkyl mit bis zu 4 Kohlenstoffatomen

oder

- für Pentafluophenyl oder
- für geradkettiges oder verzweigtes Alkyl mit bis zu 6 Kohlenstoffatomen steht, das substituiert sein kann durch Fluor, Chlor oder Phenyl

und

X - für -O-, $-OCH_2$, $-CH_2O-$, $-OCH(CH_3)-$, $CH_2N(CH_3)-$, $-CH_2N(CH_3)CH_2CH_2-$ steht,

und deren Salze.

Beispielsweise seien folgende substituierte Phenylsulfonamide genannt:

N-[4-(Chinolin-8-yloxy)phenyl]-4-chlorbenzolsulfonamid
N-[4-(Chinolin-8-yloxy)phenyl]-3-trifluormethylbenzolsulfonamid
N-[4-(Chinolin-8-yloxy)phenyl]butansulfonamid
N-[4- (Chinolin-8-yloxy)phenyl]-3-chlorpropansulfonamid
N-[4-(Chinolin-8-yloxy)phenyl]-4-fluorbenzolsulfonamid
N-[4-(Chinolin-7-yloxy)phenyl]-4-chlorbenzolsulfonamid
N-[4-(Chinolin-7-yloxy)phenyl]-3-trifluormethylbenzolsulfonamid
N-[4-(Chinolin-7-yloxy)phenyl]butansulfonamid
N-[4-(Chinolin-7-yloxy)phenyl]-3-chlorpropansulfonamid
N-[4-(Chinolin-7-yloxy)phenyl]-4-fluorbenzolsulfonamid
N-[4- (4-Methylchinolin-8-yloxy)phenyl]-4-chlorbenzolsulfonamid
N-[4-(4-Methylchinolin-8-yloxy)phanyl]-3-trifluormethylbenzolsulfonamid
N-[4-(4-Methylchinolin-8-yloxy)phenyl]butansulfonamid
N-[4-(Chinolin-8-yloxy)-3-chlorphanyl]-4-chlorbenzolsulfonamid
N-[4-(Chinolin-8-yloxy)-3-chlorphanyl]-3-trifluormethylbenzolsulfonamide
N-[4-(6-Methylchinolin-8-yloxy)phenyl]butansulfonamid
N-[4-(6-Methylchinolin-8-yloxy)phenyl]-4-chlorbenzolsulfonamid
N-[4-(Chinolin-6-yloxy)phenyl]-4-chlorbenzolsulfonamid
N-[4-(Chinolin-6-yloxy)phenyl]-3-trifluormethylbenzolsulfonamid
N-[4-(Chinolin-6-yloxy)phenyl]butansulfonamide
N-[4-(4-Methylchinolin-2-yloxy)phenyl]-4-chlorbenzolsulfonamide
N-[4-(Chinolin-2-yl-methyloxy)phenly]-4-chlorbenzolsulfonamid
N-[4-(Chinolin-2-yl-methyloxy)phenyl]-3-trifluormetphylbenzolsulfonamid
N-[4-(Chinolin-2-yl-methyloxy)phenyl]butansulfonamid
N-[4-(Chinolin-2-yl-methyloxy)phenyl]-3-chlorpropansulfonamid
N-[4-(Chinolin-2-yl-methyloxy)phenyl]pentafluorbenzolsulfonamid
N-[4-(Chinolin-2-yl-methyloxy)phenyl]-1-methylbutansulfonamid
N-(4-[l-(Chinolin-2-yl)ethyloxy]phenyl)butansulfonamid
N-[4-(Chinolin-2-yl)methyloxy-3-cyano-phenyl]butansulfonamid.

Weiterhin wurde ein Verfahren zur Herstellung der erfindungsgemäßen substituierten Phenylsulfonamide der allgemeinen Formel (I)

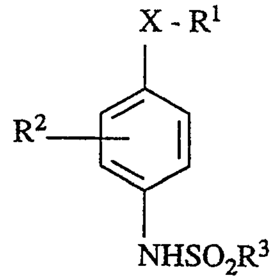

(I)

in welcher
$R^1$, $R^2$, $R^3$ und X die oben angegebene Bedeutung haben,
und deren Salze gefunden,
das dadurch gekennzeichnet ist, daß man Amine der allgemeinen Formel II

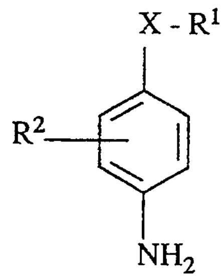

(II)

in welcher
$R^1$, $R^2$ und X die oben angegebene Bedeutung haben, mit Sulfonsäurehalogeniden der allgemeinen Formel (III)

$$R^3\text{-}SO_2\text{-}Y \quad (III)$$

in welcher
$R^3$- die oben angegebene Bedeutung hat,
und
Y - für Halogen steht,
in Gegenwart eines inerten Lösemittels, gegebenenfalls in Gegenwart einer Base umsetzt und dann gegebenenfalls im Fall der Herstellung der Salze mit einer entsprechenden Säure umsetzt.

Das erfindungsgemäße Verfahren kann durch folgendes Formelschema erläutert werden:

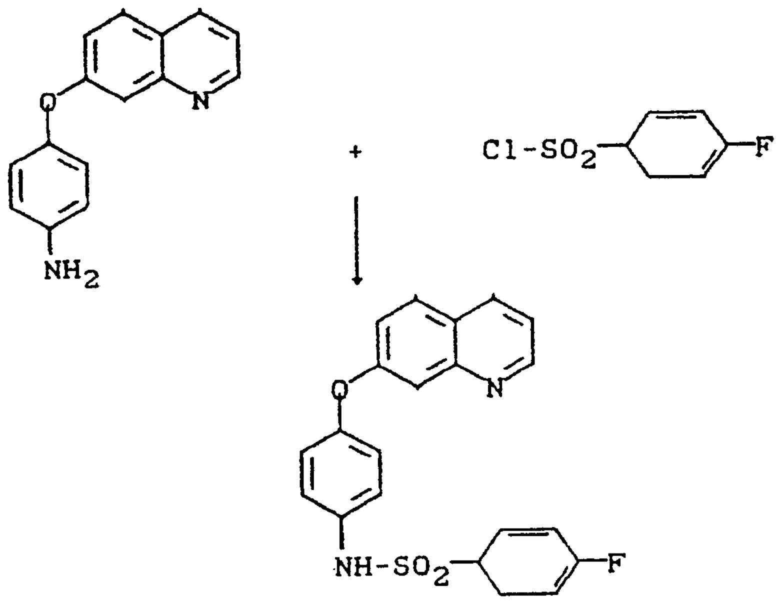

Als Lösemittel für das erfindungsgemäße Verfahren eignen sich übliche organische Lösemittel, die sich unter den Reaktionsbedingungen nicht verändern. Hierzu gehören bevorzugt Ether wie Diethylether, Dioxan, Tetrahydrofuran oder Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphaphorsäuretriamid, Acetonitril, Aceton oder Nitromethan. Ebenso ist es möglich, Gemische der genannten Lösemittel zu verwenden.

Basen für das erfindungsgemäße Verfahren können übliche basische Verbindungen sein. Hierzu gehören vorzugsweise Alkali- oder Erdalkalihydroxide wie Lithiumhydroxid, Natriumhydroxid, Kaliumhydroxid oder Bariumhydroxid, oder Alkalihydride wie Natriumhydrid, oder Alkali- oder Erdalkalicarbonate wie Natriumcarbonat, Natriumhydrogencarbonat, Kaliumcarbonat oder Calciumcarbonat, oder Alkalialkoholate wie beispielsweise Natriummethanolat, Natriumethanolat, Kaliummethanolat, Kaliumethanolat oder Kaliumtert-butylat, oder Alkaliamide wie Natriumamid oder Lithiumdiisopropylamid, oder organische Amine wie Benzyltrimethylammoniumhydroxid, Tetrabutylammoniumhydroxid, Pyridin, Dimethylaminopyridin, Triethylamin, N-Methylpiperidin, 1,5-Diazabicylo[4,3,0]non-5-en oder l,5-Diazabicyclo[5,4,0]undec-5-en.

Das erfindungsgemäße Verfahren wird im allgemeinen in einem Temperaturbereich von -30 °C bis +150 °C, bevorzugt von -20 °C bis +80 °C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Im allgemeinen setzt man 1 bis 5 Mol, bevorzugt 1 bis 2 Mol, besonders bevorzugt 1 Mol Sulfonsäurehalogenid, bezogen auf 1 Mol des Amins ein. Die Base wird im allgemeinen in einer Menge von 1 bis 10 Mol, bevorzugt von 1 bis 5 Mol, bezogen auf das Sulfonsäurehalogenid eingesetzt.

Als Sulfonsäurehalogenide für das erfindungsgemäße Verfahren seien beispielsweise genannt:
4-Toluol-sulfonylchlorid
4-Chlorphenyl-sulfonylchlorid
4-Fluorphenyl-sulfonylchlorid
3-Trifluormethylphenyl-sulfonylchlorid
Pentafluorphenyl-sulfonylchlorid
2,5-Dichlorphenyl-sulfonylchlorid
4-Methoxyphenyl-sulfonylchlorid
Propyl-sulfonylchlorid
Butyl-sulfonylchlorid
Isobutyl-sulfonylchlorid
1-Methylbutyl-sulfonylchlorid
3-Chlorpropyl-sulfonylchlorid
4-Chlorbutyl-sulfonylchlorid

Pentyl-sulfonylchlorid

Die als Ausgangsstoffe eingesetzten Amine der allgemeinen Formel (II)

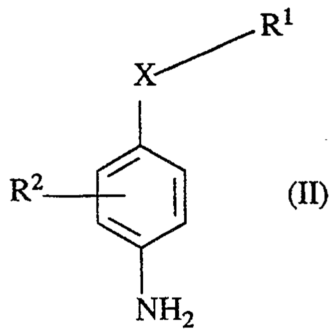

(II)

in welcher
$R^1$, $R^2$ und X die oben angegebene Bedeutung haben.
können hergestellt werden, indem man
Nitroverbindungen der allgemeinen Formel (IV)

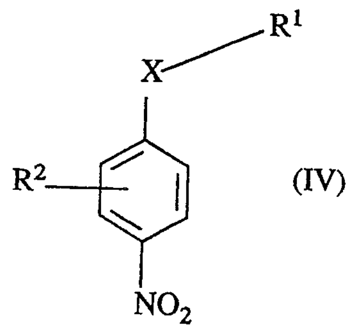

(IV)

in welcher
$R^1$, $R^2$ und X die oben angegebene Bedeutung haben reduziert.
Das erfindungsgemäße Verfahren kann durch folgendes Formelschema erläutert werden:

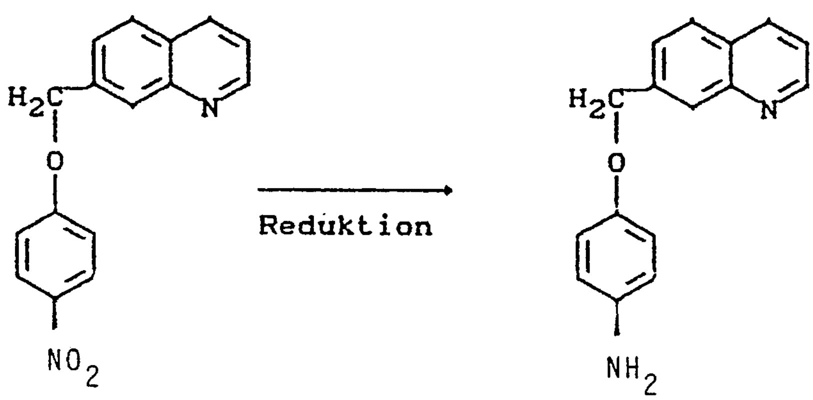


Die Reduktion erfolgt im allgemeinen durch Hydrierung mit Metallkatalysatoren wie beispielsweise Platin, Palladium, Palladium auf Tierkohle, Platinoxid oder Raney-Nickel, bevorzugt mit Palladium auf Tierkohle, in Anwesenheit von Säuren.

Als Säuren können erfindungsgemäß starke Mineralsäuren aber auch organische Säuren eingesetzt werden. Bevorzugt sind dies Salzsäure, Bromwasserstoffsäure, Schwefelsäure, Phosphorsäure, oder Carbonsäuren wie Essigsäure, Oxalsäure, Trifluoressigsäure, oder Sulfonsäuren wie Methan-, Ethan-, Phenyl- oder Toluolsulfonsäure, oder Naphthalindisulfonsäure.

Der Katalysator wird hierbei im allgemeinen in einer Menge von 0,1 bis 50 Mol-%, bevorzugt von 1 bis 10 Mol-% bezogen auf 1 Mol der Nitroverbindung eingesetzt.

Die Hydrierung erfolgt im allgemeinen in einem Temperaturbereich von -20° C bis +100° C, bevozugt im Bereich von 0° C bis +50° C.

Im allgemeinen erfolgt die Hydrierung bei Normaldruck. Es ist ebenso möglich, die Hydrierung bei einem Überdruck von 2 bis 200 bar, bevorzugt von 2 bis 50 bar durchzuführen.

Als Lösemittel für die Hydrierung eignen sich Wasser und inerte organische Lösemittel. Bevorzugt gehören hierzu Alkohole wie beispielsweise Methanol, Ethanol, Propanol oder Isopropanol, oder Ether wie Diethylether, Dioxan, Tetrahydrofuran, Glykolmono- oder -dimethylether, oder Chlorkohlenwasserstoffe wie Methylenchlorid, Chloroform oder Tetrachlorkohlenstoff, oder Eisessig, Trifluoressigsäure, Dimethylformamid, Hexamethylphosphorsäuretriamid, Essigester, Aceton oder Pyridin. Ebenso ist es möglich, Gemische der genannten Lösemittel einzusetzen.

Darüberhinaus kann die Reduktion auch nach Methoden durchgeführt werden, wie sie im allgemeinen zur Reduktion von Nitrogruppen zu Aminogruppen üblich ist. Hierbei seien beispielsweise zu nennen: Die Reduktion mit Hydrazin in Wasser und/oder Alkoholen wie beispielsweise Methanol, Ethanol, Propanol oder Isopropanol, bevorzugt in Anwesenheit von Katalysatoren wie Platin, Palladium oder Palladium auf Tierkohle in einem Temperaturbereich von 0° C bis +150° C, bevorzugt von +20° C bis +100° C.

Die Reduktion mit Lithiumaluminiumhyrid in inerten Lösemitteln wie Ether z.B. Diethylether, Dioxan oder Tetrahydrofuran, oder Kohlenwasserstoffe wie Benzol. Toluol oder Xylol, oder Chlorkohlenwasserstoffe wie Methylenchlorid, Chloroform oder Tetrachlorkohlenstoff in einem Temperaturbereich von -30° C bis +150° C, bevorzugt von 0° C bis +80° C oder Reduktion mit Zink in Wasser und/oder Alkoholen wie Methanol, Ethanol, Propanol oder Isopropanol in Anwesenheit von Säuren wie Salzsäure oder Essigsäure.

Ebenso können die erfindungsgemäß verwendeten Amine hergestellt werden, wie es beispielsweise in DE-A-1 36 07 382 beschrieben wird.

Als Amine werden beispielsweise erfindungsgemäß verwendet:

7-(4-Aminophenoy)chinolin
8-(4-Aminophenoxy)chinolin
8-(4-Aminophenoxy)-4-methyl-chinolin
8-(4-Amino-2-chlorphenoxy)chinolin
8-(4-Aminophenoxy)-6-methyl-chinolin
2-(4-Aminophenoxymethyl)chinolin
2-(4-Amino-2-cyano-phenoxymethyl)chinolin
2-[1-(4-Aminophenoxy)ethyl]chinolin
2-(4-Amino-2-ethoxycarbonyl-phenoxymethyl)chinolin

Die als Ausgangsstoffe eingesetzten Nitroverbindungen der allgemeinen Formel (IV), wobei

a) Nitroverbindungen, in welchen $R^1$ und $R^2$ die angegebene Bedeutung haben und X für -O- steht der allgemeinen Formel (IVa)

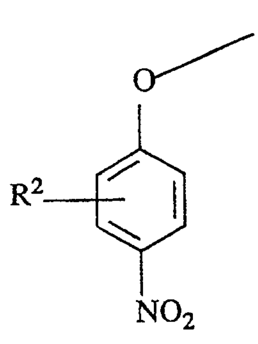

(IVa)

entsprechen und wobei

b) Nitroverbindungen,in welchen $R^1$ und $R^2$ die angegebene Bedeutung haben und X für -A-B- steht, der allgemeinen Formel (IVb)

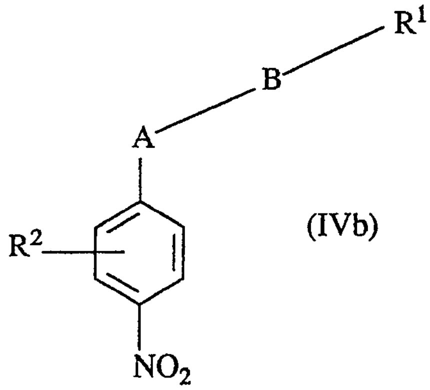

(IVb)

entsprechen und wobei

c) Nitroverbindungen in welchen $R^1$ und $R^2$ die angegebene Bedeutung haben und X für -B-A steht, der allgemeinen Formel (IVc)

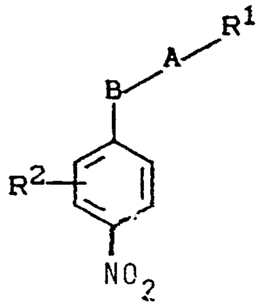

(IVc)

entsprechen,

können hergestellt werden, indem man

Fluornitrophenylverbindungen der allgemeinen Formel (V)

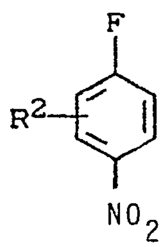

(V)

in welcher $R^2$ die angegebene Bedeutung hat,

mit Alkoholen der allgemeinen Formel (VI)

$$R^1\text{-OH} \quad (VI)$$

in welcher $R^1$ die angegebene Bedeutung hat,

in geeigneten Lösemitteln in Anwesenheit von Basen umsetzt.

Die Reaktion kann durch folgendes Formelschema verdeutlicht werden:

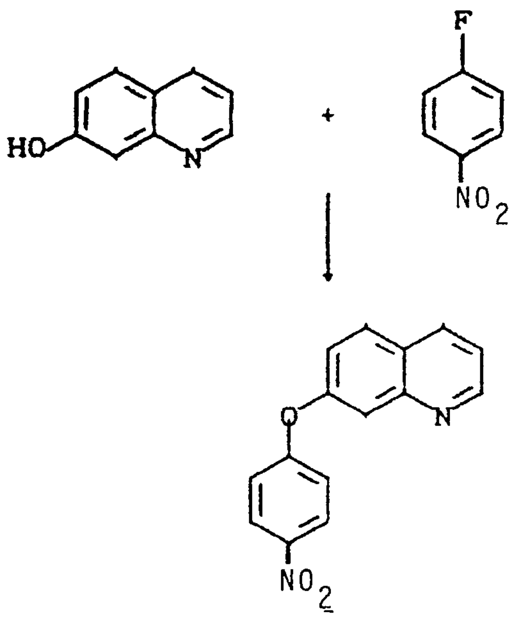

Als Lösemittel eignen sich die üblichen organischen Lösemittel, die sich unter den Reaktionsbedingungen nicht verändern. Hierzu gehören bevorzugt Ether, wie beispielsweise Dioxan, Tetrahydrofuran oder Diethylether, oder Chlorkohlenwasserstoffe wie Methylenchlorid, Trichlormethan oder Tetrachlormethan, oder Kohlenwasserstoffe wie Benzol, Toluol, Xylol, Hexan, Cyclohexan, oder Erdölfraktionen, oder Amide wie Dimethylformamid oder Hexamethylphosphorsäuretriamid, oder Pyridin. Ebenso ist es möglich, Gemische der genannten Lösemittel einzusetzen.

Als Basen können die üblichen anorganischen oder organischen Basen eingesetzt werden. Hierzu gehören vorzugsweise Alkalihydroxide wie beispielsweise Natriumhydroxid oder Kaliumhydroxid, oder Erdalkalihydroxide wie beispielsweise Bariumhydroxid, oder Alkalicarbonate wie Natriumcarbonat oder Kaliumcarbonat, oder Erdalkalicarbonate wie Calciumcarbonat, oder organische Amine wie Triethylamin, Pyridin oder Methylpiperidin.

Das Verfahren wird im allgemeinen in einem Temperaturbereich von 0° C bis +150° C, bevorzugt von +20° C bis +100° C durchgeführt.

Das Verfahren wird im allgemeinen bei Normaldruck durchgeführt, es ist aber auoh möglich das Verfahren bei Unterdruck oder bei Überdruck durchzuführen (z.B. in einem Bereich van 0,5 bis 5 bar).

Im allgemeinen setzt man 0,5 bis 2 Mol, bevorzugt 1 Mol Alkohol bezogen auf 1 Mol Fluornitrophenylverbindung ein.

Als Fluornitrophenylverbindung wird beispielsweise erfindungsgemäß verwendet:
4-Fluornitrobenzol.

Als Alkohole werden beispielsweise erfindungsgemäß verwendet:
2-Hydroxychinolin,
4-Hydroxychinolin,
5-Hydroxychinolin,
8-Hydroxychinolin,
1-Hydroxyisochinolin,
5-Hydroxyisochinolin,
2-Hydroxy-4-methyl-chinolin,
8-Hydroxy-4-methyl-chinolin,
8-Hydroxy-6-methyl-chinolin.

Die als Ausgangsstoffe verwendeten Verbindungen der allgemeinen Formel (V) und (VI) sind bekannt.

Die Verbindungen der allgemeinen Formel (IVb) werden hergestellt,
indem man
Nitrophenylverbindungen der allgemeinen Formel (VII)

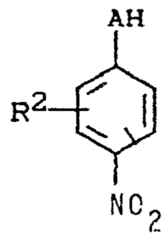

(VII)

in welcher
$R^2$ und A die angegebene Bedeutung haben,
mit Halogeniden der allgemeinen Formel (VIII)

$$Hal\text{-}B\text{-}R^1 \qquad (VIII)$$

in welcher
$R^1$ und B die angegebene Bedeutung haben
und
Hal - für Chlor, Brom oder lod steht,
in geeigneten Lösemitteln, gegebenenfalls in Anwesenheit einer Base umsetzt.

Das erfindungsgemäße Verfahren kann beispielsweise durch das folgende Formelschema erläutert werden:

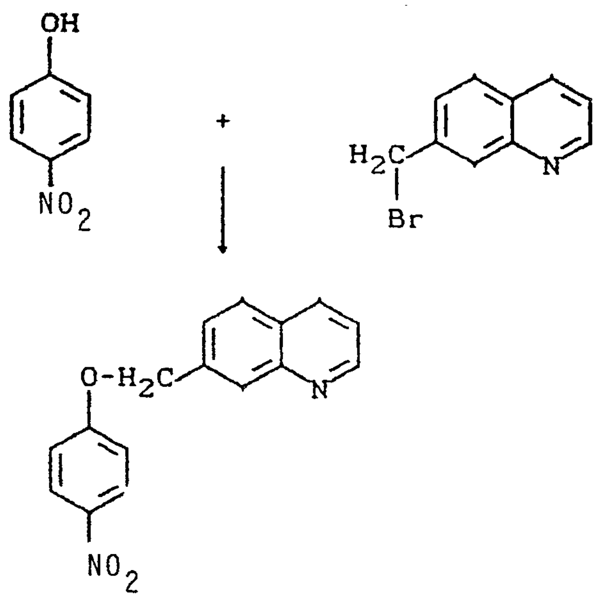

Als Lösemittel eignen sich die üblichen organischen Lösemittel, die sich unter den Reaktionsbedingungen nicht verändern. Hierzu gehören vorzugsweise Alkohole wie beispielsweise Methanol, Ethanol, Propanol oder Isopropanol, oder Ether wie beispielsweise Dioxan, Tetrahydrofuran oder Diethylether, oder Chlorkohlenwasserstoffe wie beispielsweise Dichlormethan, Trichlormethan, Tetrachlormethan, 1,2-Dichlorethan oder Trichlorethylen, oder Kohlenwasserstoffe wie Benzol, Xylol, Toluol, Hexan, Cyclohexan, oder Erdölfraktionen, oder Nitromethan, Dimethylformamid, Acetonitril, Aceton oder Hexamethylphosphorsäuretriamid. Ebenso ist es möglich, Gemische der genannten Lösemittel einzusetzen.

Als Basen eignen sich anorganische oder organische Basen. Hierzu gehören vorzugsweise Alkalihydroxide wie beispielsweise Natriumhydroxid oder Kaliumhydroxid, oder Erdalkalihydroxide wie Bariumhydroxid, oder Alkalicarbonate wie beispielsweise Natriumcarbonat oder Kaliumcarbonat, oder Erdalkalicarbonate wie Calciumcarbonat oder organische Amine wie beispielsweise Triethylamin, Pyridin, Methylpiperidin, Piperidin oder Morpholin.

Es ist auch möglich, als Basen Alkalimetalle wie Natrium, oder deren Hydride wie Natriumhydrid einzusetzen.

Das Verfahren wird im allgemeinen in einem Temperaturbereich von 0°C bis +150°C, bevorzugt von +10°C bis +100°C durchgeführt.

Das Verfahren wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 Mol Halogenid bezogen auf 1 Mol Nitrophenylverbindung ein. Die Base wird im allgemeinen in einer Menge von 0,5 bis 5 Mol, bevorzugt von 1 bis 3 Mol, bezogen auf das Halogenid eingesetzt.

Als Nitrophenylverbindung wird beispielsweise erfindungsgemäß verwendet:
4-Nitrophenol.

Als Halogenide werden beispielsweise erfindungsgemäß verwendet:
8-Chlormethyl-chinolin,
7-Chlormethyl-chinolin,
2-Chlormethyl-chinolin,
2-Chlormethyl-4-methyl-chinolin,
8-Chlormethyl-6-methyl-chinolin.

Die Ausgangsverbindungen (VII) und (VIII) sind bekannt.

Die Verbindungen der allgemeinen Formel (IVc) werden hergestellt indem man

Verbindungen der allgemeinen Formel (IX)

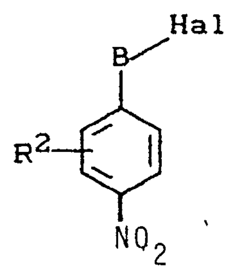

(IX)

in welcher
$R^2$ und B die oben angegebene Bedeutung haben
und
Hal - für Chlor, Brom oder Iod steht,
mit Verbindungen der allgemeinen Formel (X)

$$H\text{-}A\text{-}R^1 \qquad (X)$$

in welcher
A und $R^1$ die oben angegebene Bedeutung haben,
in geeigneten Lösemitteln, gegebenenfalls in Anwesenheit einer Base umsetzt.

Das Verfahren kann beispielsweise durch das folgende Formelschema erläutert werden:

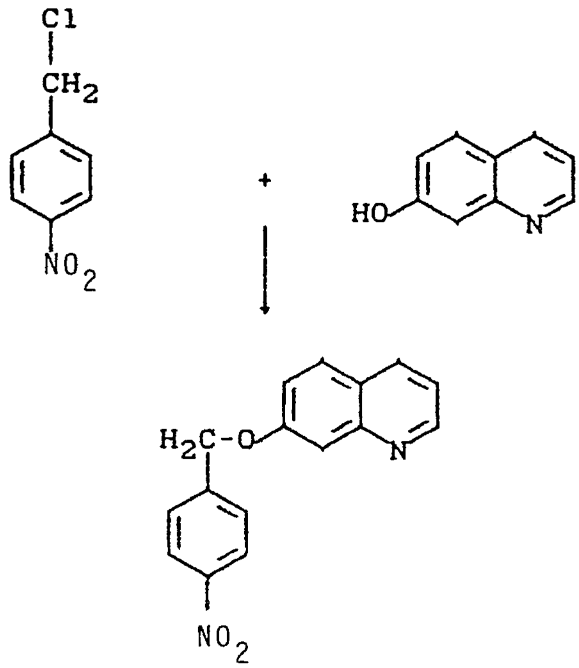

Lösemittel, Basen sowie die Bedingungen zur Durchführung des Verfahrens zur Herstellung der Verbindungen der allgemeinen Formel (IVc) wurden bereits ausführlich für das Verfahren zur Herstellung der Verbindungen der allgemeinen Formel (IVb) beschrieben.

Als Halogenid wird beispielsweise erfindungsgemäß verwendet:
4-Chlormethyl-nitrophenol.

Als Hydroxyverbindungen wurden beispielsweise erfindungsgemäß verwendet:
2-Hydroxy-chinolin,
3-Hydroxy-chinolin,
5-Hydroxy-chinolin,
6-Hydroxy-chinolin,
8-Hydroxy-chinolin,
5-Hydroxy-4-methyl-chinolin,
8-Hydroxy-4-methyl-chinolin.

Die als Ausgangsstoffe verwendeten Verbindungen der allgemeinen Formel (IX) und (X) sind bekannt.

Die erfindungsgemäßen substituierten Phenylsulfonamide können als Wirkstoffe in Arzneimitteln eingesetzt werden. Die neuen Stoffe wirken als Hemmer (Stimulatoren) von enzymatischen Reaktionen im Rahmen des Arachidonsäurestoffwechsels, insbesondere der Lipoxygenase. Darüberhinaus wirken sie thrombozytenaggregationshemmend.

Sie sind somit bevorzugt zur Behandlung und Verhütung von Erkrankungen der Atemwege wie Allergien/Asthma, Bronchitis, Emphysem, Schocklunge, Pulmonarer Hypertonie, Entzündungen, Rheuma, Ödemen, Thrombosen, Thromboembolien, Ischämien (periphere, cardiale, cerebrale Durchblutungsstörungen), Herz- und Hirninfarkten, Herzrhythmusstörungen, Angina Pectoris, Arteriosklerose, bei Gewebstransplantationen, Dermatosen wie Psoriasis, Metastasen und zur Cytoprotektion im GastrointestinalTrakt geeignet.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-FettalkoholEther, Alkylsulfonate, Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in'Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Die erfindungsgemäßen substituierten Phenylsulfonamide können sowohl in der Humanmedizin als auch in der Veterinärmedizin eingesetzt werden.

Herstellungsbeispiele

Die Retentionszeiten $R_t$ (min) werden mit einem HPLCGerät (Fa. Knauer) an Hibar-Säulen (Fa. Merck) ermittelt.

System a: RP-8, 7 um
Durchfluß: 2 ml/min
Eluens: Acetonitril/Wasser = 70:30 (v/v)

Beispiel 1

8-(2-Nitrophenoxy)chinolin

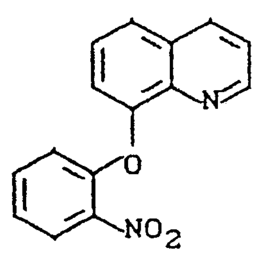

29 g 8-Hydroxychinolin und 28 g wasserfreies Kaliumcarbonat werden 1 h bei 25 °C in 400 ml Dimethylformamid gerührt. Es werden 21 ml 2-Fluor-nitrobenzol, gelöst in 100 ml Dimethylformamid, zugetropft, und das Reaktionsgemisch wird 15 h bei 25 °C gerührt. Das Lösungsmittel wird im Vakuum abgedampft, der Rückstand in Essigester aufgenommen und dreimal mit Wasser gewaschen. Nach Trocknen über Natriumsulfat wird das Lösungsmittel im Vakuum abgedampft und der Rückstand aus Methanol umkristallisiert.
Ausbeute: 82% der Theorie

Fp. : 113 - 114° C (Methanol)

Analog Beispiel 1 wurden hergestellt:

Beispiel 2

8-(4-Nitrophenoxy)chinolin

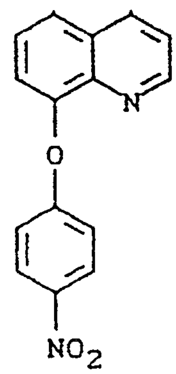

Ausbeute: 80%
Fp. : 165 - 166° C (Methanol)

Beispiel 3

4-Methyl-8-(4-nitrophenoxy)chinolin

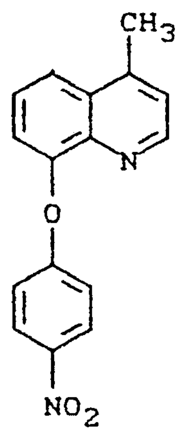

Ausbeute: 88% der Theorie
Fp. : 148 - 149%C (Methanol)

Beispiel 4

8-(2-Chlor-4-nitrophenoxy)chinolin

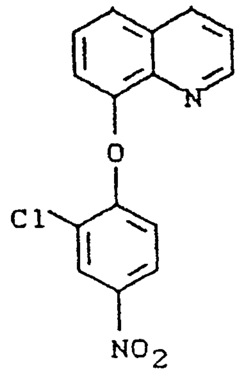

Ausbeute: 89% der Theorie
Fp. : 113 - 115° C (Ethanol)

Beispiel 5

6-Methyl-8-(4-nitrophenoxy)chinolin

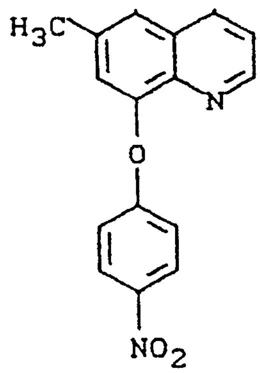

Ausbeute: 60% der Theorie
Fp. : 143° C (Ethanol)

Beispiel 6

2-(4-Nitrophenoxymethyl)chinolin

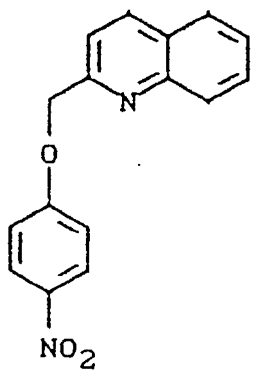

28 g 4-Nitrophenol und 55 g wasserfreies Kaliumcarbonat werden 1 h bei 25° C in 300 ml Dimethylformamid gerührt. Nach Zutropfen einer Suspension von 43 g 2-Chlorphenyl-chinolin-hydrochlorid in 100 ml Dimethylformamid wird 15 h bei 40 - 50 °C gerührt. Nach Abdampfen des Lösemittels wird der Rückstand mit Wasser verrührt, abgesaugt und aus Methanol umkristallisiert.
Ausbeute: 91% der Theorie
Fp. : 144 - 145 °C (Methanol)

Analog Beispiel 6 wurden hergestellt:

Beispiel 7

2-(2-Cyano-4-nitrophenoxymethyl)chinolin

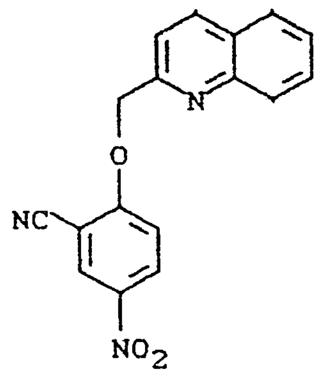

Ausbeute: 50% der Theorie
Fp. : 161 - 162 °C (Methanol)

Beispiel 8

2-[1-(4-Nitrophenoxy)ethyl]chinolin

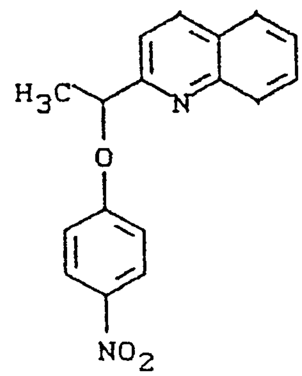

Ausbeute: 75% der Theorie
$R_t$ = 2.07 (System a)

Beispiel 9

2-(2-Ethoxycarbonyl-4-nitrophenoxymethyl)chinolin

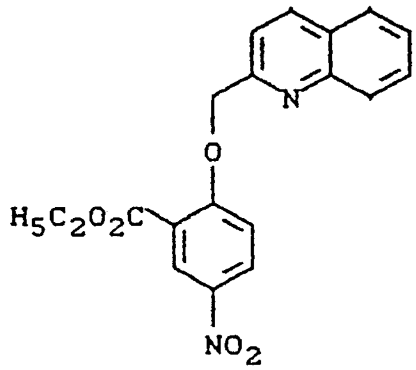

Ausbeute: 40% der Theorie
Fp. : 139 - 140° C (Ethanol)

Beispiel 10

8-(2-Nitrobenzyloxy)chinolin

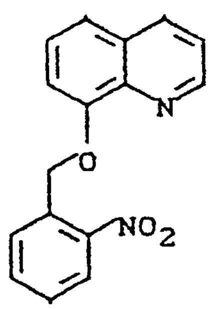

42 g 8-Hydroxychinolin und 40 g wasserfreies Kaliumcarbonat werden 1 h bei 25° C in 400 ml Dimethylformamid gerührt. Danach werden 50 g 2-Nitrobenzylchlorid in 150 ml Dimethylformamid zugetropft, die Mischung 15 h bei 25° C gerührt und eingedampft. Der Rückstand wird mit Wasser verrührt, abgesaugt und aus Ethanol umkrisallisiert. Ausbeute: 84% der Theorie
Fp. : 151 - 153° C (Ethanol)

Analog Beispiel 10 wurden hergestellt:

Beispiel 11

8-(2-Aminophenoxy)chinolin

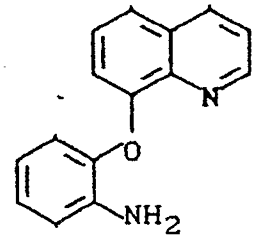

35,4 g 8-(2-Nitrophenoxy)chinolin und 3,4 g 10%iges Palladium/Kohle werden unter Stickstoff in 350 ml Methanol suspendiert und erwärmt. Unter Rückfluß werden 27,8 ml Hydrazinhydrat langsam zugetropft, danach wird weitere 2 h im Rückfluß erhitzt. Nach Abkühlen wird der Katalysator abfiltriert und das

Lösemittel im Vakuum abgedampft. Der Rückstand wird aus Methanol umkristallisiert.
Ausbeute: 69% der Theorie
Fp. : 135° C (Methanol)

Analog Beispiel 11 wurden hergestellt:

Beispiel 12

7-(4-Aminophenoxy)chinolin

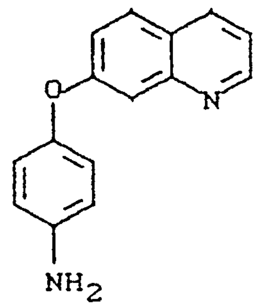

Ausbeute: 72% der Theorie
Fp. : 131° C (Ethanol)

Beispiel 13

8-(4-Aminophenoxy)chinolin

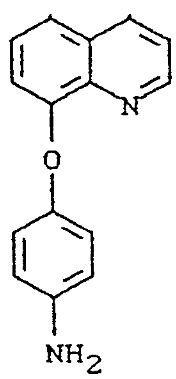

Ausbeute: 68% der Theorie
Fp. : 204° C (Methanol)

Beispiel 14

8-(4-Aminophenoxy)-4-methyl-chinolin

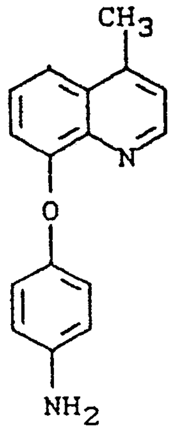

Ausbeute: 7l% der Theorie
Fp. : 157 - 159°C (Ethanol)

Beispiel 15

8(4-Amino-2-chlorphenoxy)chinolin

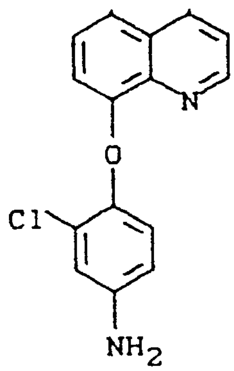

Ausbeute: 28% der Theorie
Fp. : 181 - 182°C

Beispiel 16

8-(4-Aminophenoxy)-6-methyl-chinolin

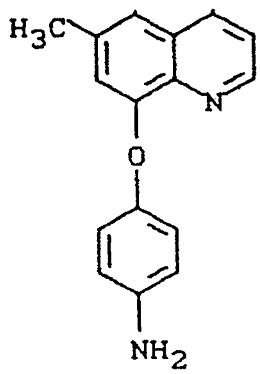

Ausbeute: 77% der Theorie
Fp. : 184 - 185 °C (Ethanol)

Beisniel 17

2-(4-Aminophenoxymethyl)chinolin

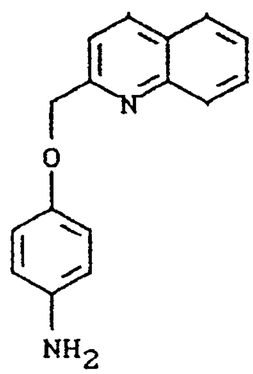

Ausbeute: 64% der Theorie
Fp. : 126 - 128 °C (Methanol)

Beispiel 18

2-(4-Amino-2-cyano-phenoxymethyl)chinolin

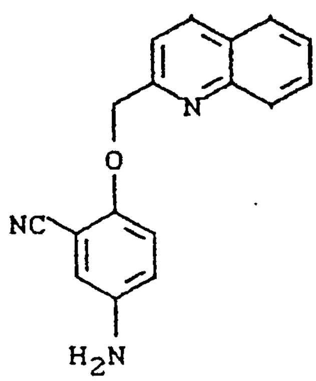

Ausbeute: 49% der Theorie
Fp. : 156° C

Beispiel 19

2-[1-(4-Aminophenoxy)ethyl]chinolin

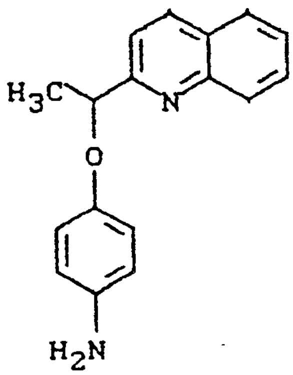

Ausbeute: 95% der Theorie
Fp. : 86 - 88° C

Beispiel 20

2-(4-Amino-2-ethoxycarbonyl-phenoxymethyl)chinolin

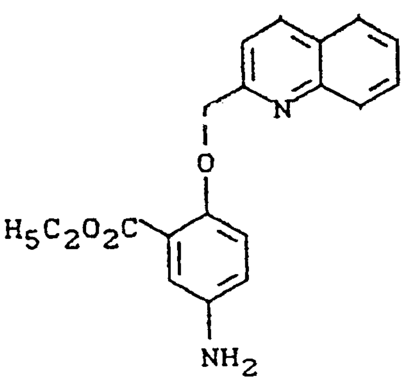

Ausbeute: 57% der Theorie
Fp. : 93 - 95° C

Beispiel 21

N-[2-(Chinolin-8-yloxy)phenyl]-4-chlorbenzolsulfonamid

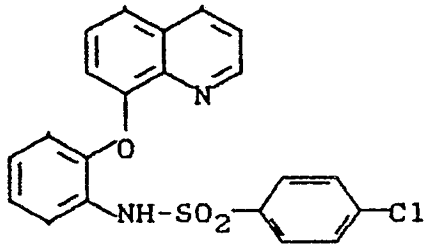

3,54 g 8-(2-Aminophenoxy)chinolin werden in 70 ml Dichlormethan gelöst und bei 25° C mit einer Lösung von 3,17 g 4-Chlorbenzolsulfonsäurechlorid in 30 ml Dichlormethan versetzt. Nach 1 h werden 2.42 ml Pyridin zugegeben und 15 h bei 25° C gerührt. Nach Abdampfen des Lösemittels wird der Rückstand mit Wasser verrührt. Das Produkt wird abfiltriert und aus Ethanol umkristallisiert.
Ausbeute: 94% der Theorie
Fp. : 135 - 137° C (Ethanol)

Analog Beispiel 21 wurden hergestellt:

Beispiel 22

N-[4-(Chinolin-8-yloxy)phenyl]-4-chlorbenzolsulfonamid

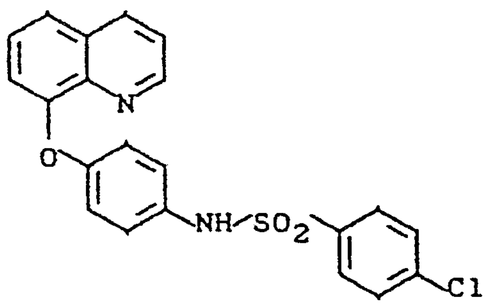

Ausbeute: 91% der Theorie
Fp. : 220° C (Methanol)

Beispiel 23

N-[4-(Chinolin-8-yloxy)phenyl]-3-trifluormethylbenzol-sulfonamid

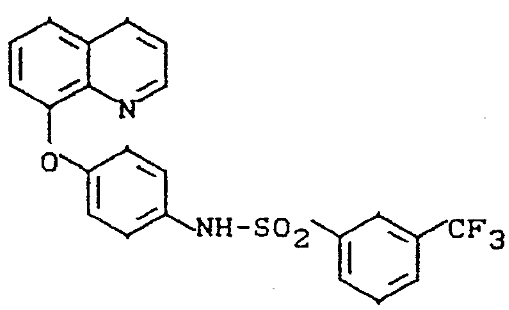

Ausbeute: 66% der Theorie
Fp. : 186° C (Methanol)

Beispiel 24

N-[4-(Chinolin-8-yloxy)phenyl]butansulfonamid

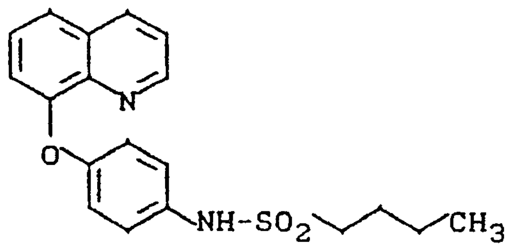

Ausbeute: 65% der Theorie
Fp. : 162° C (Methanol)

Beispiel 25

N-[4-(Chinolin-8-yloxy)phenyl]-3-chlorpropansulfonamid

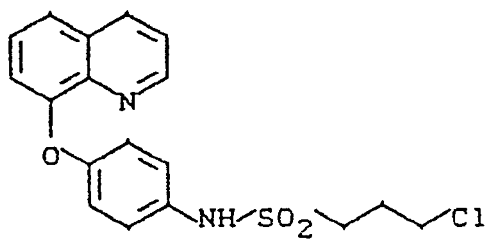

Ausbeute: 69% der Theorie
Fp. : 161 - 162° C (Methanol)

Beispiel 26

N-[4-(Chinolin-8-yloxy)phenyl]-4-fluorbenzolsulfonamid

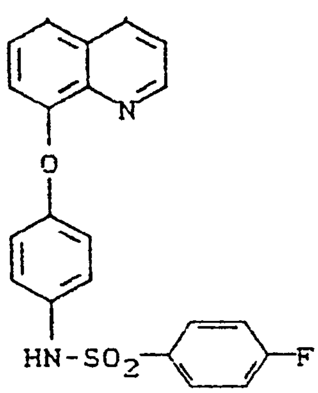

Ausbeute: 76% der Theorie
Fp. : 181 - 183° C (Methanol)

Beispiel 27

N-[4-(Chinolin-7-yloxy)phenyl]-4-chlorbenzolsulfonamid

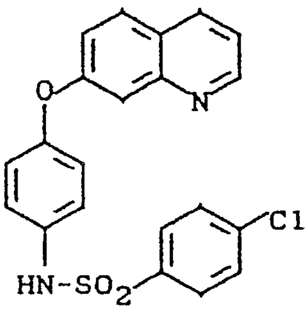

Ausbeute: 93% der Theorie
Fp. : 208° C (Methanol)

Beispiel 28

N-[4-(Chinolin-7-yloxy)phenyl]-3-trifluormethylbenzol-sulfonamid

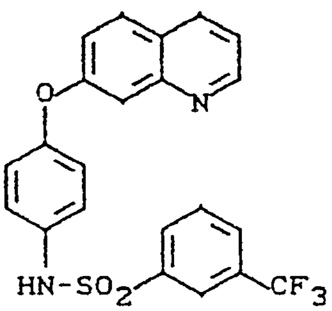

Ausbeute: 64% der Theorie
Fp. : I90° C (Methanol)

Beispiel 29

N-[4-(Chinolin-7-yloxy)phenyl]butansulfonamid

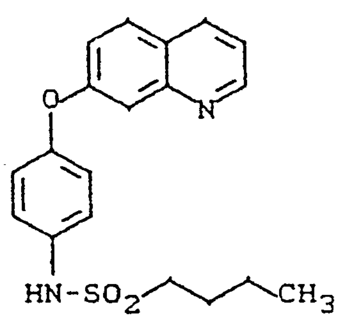

Ausbeute: 70% der Theorie
Fp. : 168° C (Methanol)

Beispiel 30

N-[4-(Chinolin-7-yloxy)phenyl]-3-chlorpropansulfonamid

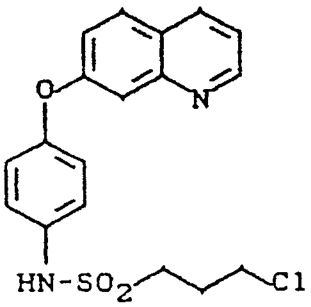

Ausbeute: 75% der Theorie
Fp. : 175 - 176° C (Methanol)

Beispiel 31

N-[4-(Chinolin-7-yloxy)phenyl]-4-fluorbenzolsulfonamid

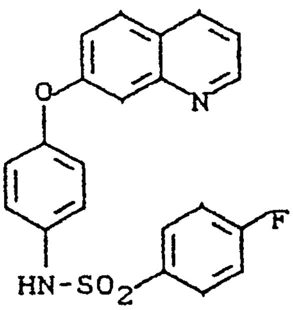

Ausbeute: 61% der Theorie
Fp. : 175 - 178° C (Methanol)

Beispiel 32

N-[4-(4-Methylchinolin-8-yloxy)phenyl]-4-chlorbenzol-sulfonamid

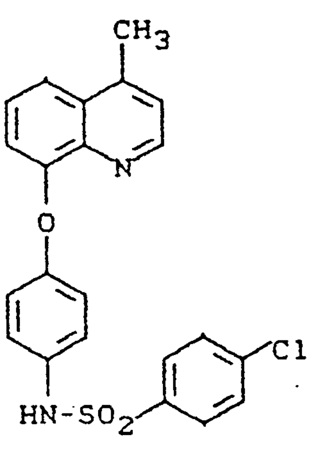

Ausbeute: 94% der Theorie
Fp. : 223 - 224 °C (Methanol)

Beispiel 33

N-[4-(4-Methylchinolin-8-yloxy)phenyl]-3-trifluormethyl-benzolsulfonamid

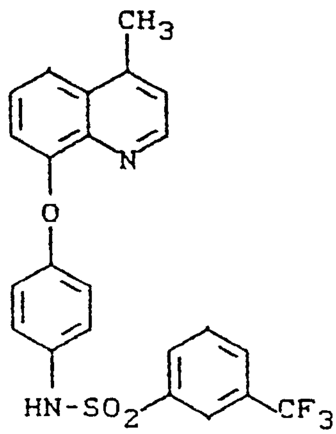

Ausbeute: 70% der Theorie
Fp. : 202 - 203 °C

Beispiel 34

N-[4-(4-Methylchinolin-8-yloxy)phenyl]butansulfonamid

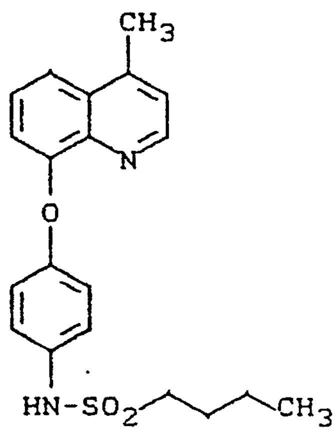

Ausbeute: 81% der Theorie
Fp. : 208 - 209 °C (Ethanol)

Beispiel 35

N-[4-(Chinolin-8-yloxy)-3-chlorphenyl]-4-chlorbenzol-sulfonamid

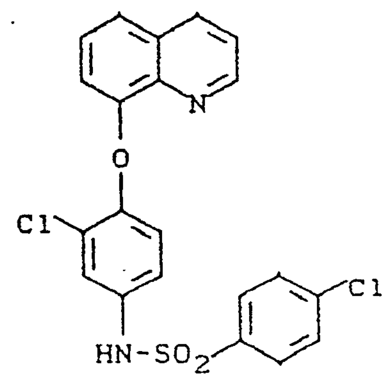

Ausbeute: 90% der Theorie
Fp. : 198 - 199 °C (Ethanol)

Beispiel 36

N-[4-(Chinolin-8-yloxy)-3-chlorphenyl]-3-trifluormethyl-benzolsulfonamide

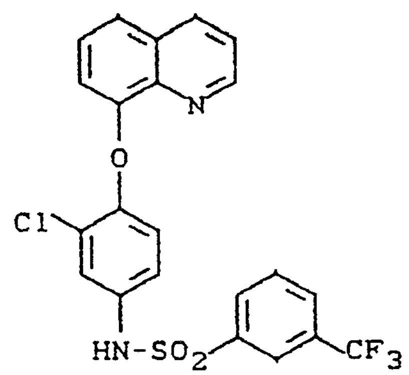

Ausbeute: 88% der Theorie
Fp. : >245 °C (Methanol)

Beispiel 37

N-[4-(6-Methylchinolin-8-yloxy)phenyl]butansulfonamid

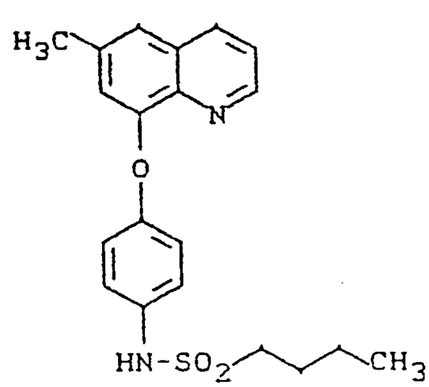

Ausbeute: 88% der Theorie
Fp. : 189 - 190 ° C (Ethanol)

Beispiel 38

N-[4-(6-Methylchinolin-8-yloxy)phenyl]-4-chlorbenzol-sulfoanmid

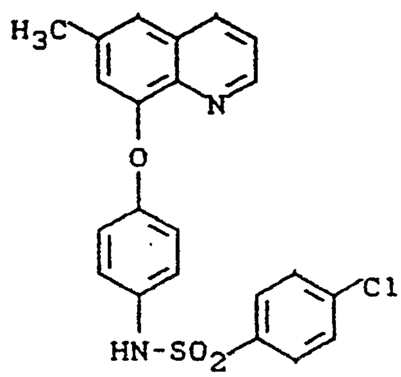

Ausbeute: 94% der Theorie
Fp. : >245 ° C

Beispiel 39

N-[4-(Chinolyl-6-yloxy)phenyl]-4-chlorbenzol-sulfonamid

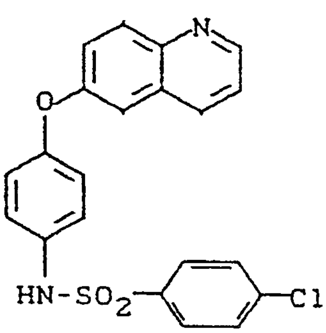

Ausbeute: 33% der Theorie
Fp. : >255 ° C

Beispiel 40

N-[4-(Chinolin-6-yloxy)phenyl]-3-trifluormethylbenzol-sulfonamid

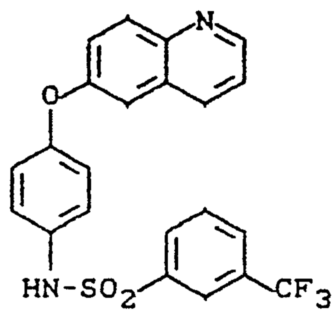

Ausbeute: 60% der Theorie
Fp. : 142 - 143° C (Methanol)

Beispiel 41

N-[4-(Chinolin-6-yloxy)phenyl]butansulfonamide

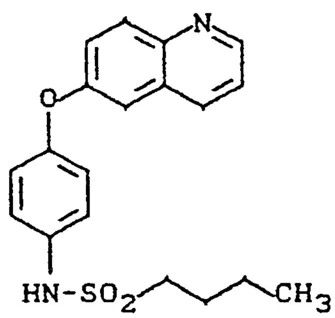

Ausbeute: 84% der Theorie
Fp. : 170° C (Methanol)

Beispiel 42

N-[4-(4-Methylchinolin-2-yloxy)phenyl]-4-chlorbenzol-sulfonamid

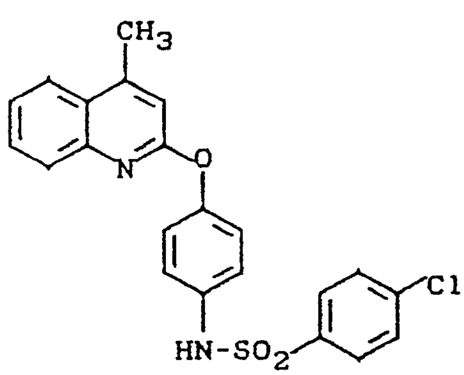

Ausbeute: 88% der Theorie
Fp. : 174 - 176° C (Methanol)

Beispiel 43

N-[4-(Chinolin-2-yl-methyloxy)phenly]-4-chlorbenzol-sulfonamid

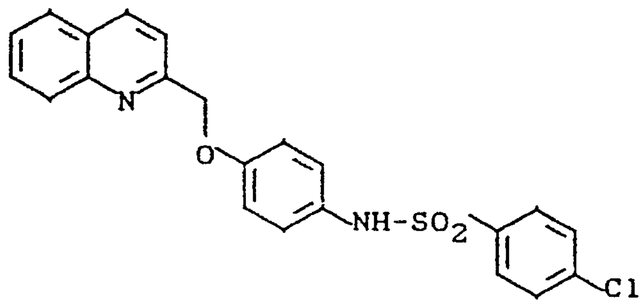

Ausbeute: 82% der Theorie
Fp. : 125° C (Methanol)

Beispiel 44

N-[4-(Chinolin-2-yl-methyloxy)phenyl]-3-trifluormethyl-benzolsulfonamid

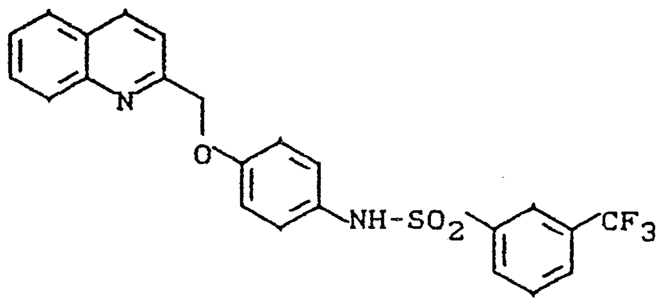

Ausbeute: 93% der Theorie
Fp. : 81 - 83° C (Methanol)

Beispiel 45

N-[4-(Chinolin-2-yl-methyloxy)phenyl]butansulfonamid

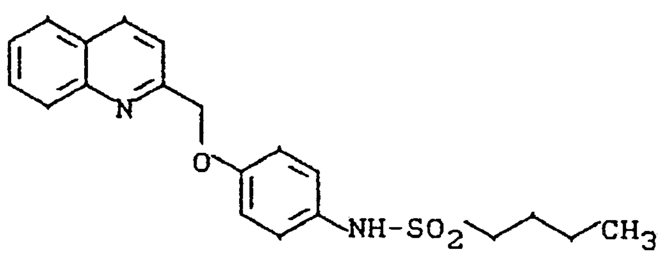

Ausbeute: 77% der Theorie
Fp. : 113° C (Ethanol)

Beispiel 46

N-[4-(Chinolin-2-yl-methyloxy)phenyl]-3-chlorpropan-sulfonamid

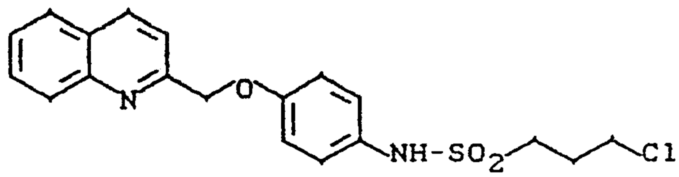

Ausbeute: 74% der Theorie
Fp. : 117° C (Methanol)

Beispiel 47

N-[4-(Chinolin-2-yl-methyloxy)phenyl]pentafluorbenzolsulfonamid

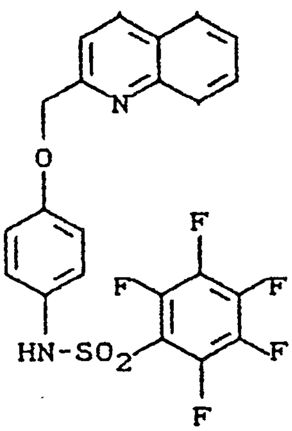

Ausbeute: 37% der Theorie
Fp. : 170 - 178° C (Toluol)

Beispiel 48

N-[4-(Chinolin-2-yl-methyloxy)phenyl]-1-methylbutan-sulfonamid

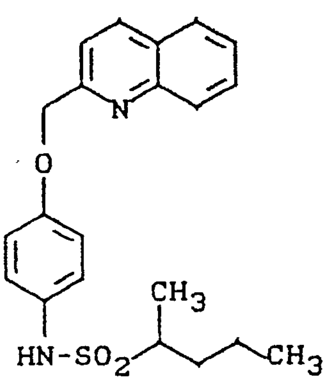

Ausbeute: 70% der Theorie
$R_t$ = 1.68 (System a)

Beispiel 49

N-{4-[1-(Chinolin-2-yl)ethyloxy]phenyl}butansulfonamid

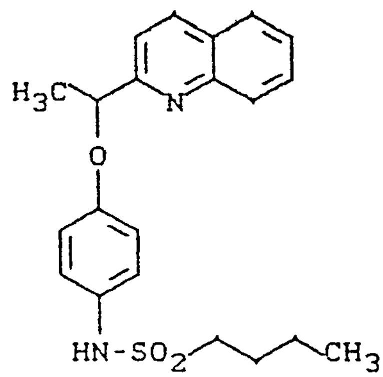

Ausbeute: 89% der Theorie
$R_t$ = 1.80 (System a)

Beispiel 50

N-[4-(Chinolin-2-yl]methyloxy-3-cyano-phenyl]butan-sulfonamid

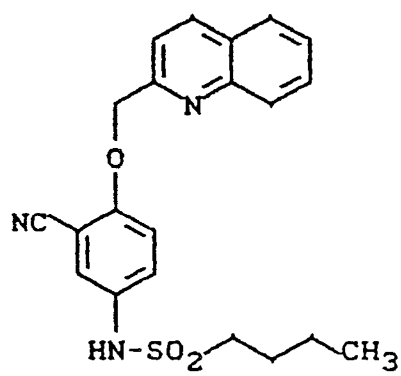

Ausbeute: 43% der Theorie
Fp. : 158 -160 ° C (Isopropanol)

Beispiel 51

N-[3-Ethoxycarbonyl-4-(chinolin-2-yl )methyloxy-phenyl]-butansulfonamid

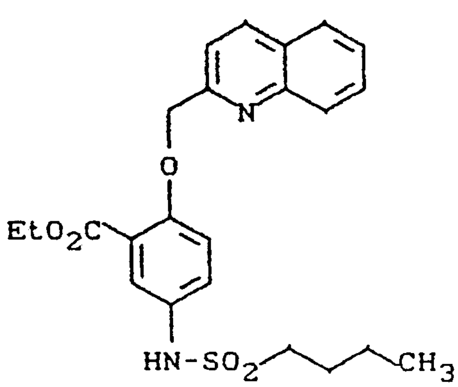

Ausbeute: 33% der Theorie
Fp. : 90 - 92° C

Anwendungsbeispiele;

(Thrombozytenaggregationshemmung)

Zur Bestimmung der thrombozytenaggregationshemmenden Wirkung wurde Blut von gesunden Probanden beiderlei Geschlechts verwendet. Als Antikoagulans wurden einem Teil 3,8%iger wäßriger Natriumzitratlösung 9 Teile Blut zugemischt. Mittels Zentrifugation erhält man aus diesem Blut plättchenreiches Zitratplasma (PRP) (Jürgens/Beller, Klinische Methoden der Blutgerinnungsanalyse; Thieme Verlag, Stuttgart, 1959).

Für diese Untersuchungen wurden 0,8 ml PRP und 0,1 ml der Wirkstofflösung bei 37° C im Wasserbad vorinkubiert. An-schließend wurde die Thrombozytenaggregation nach der turbidometrischen Methode (Born, G.V.R., J. Physiol. (London ), 162, 67, 1962) im Aggregometer bei 37° C bestimmt (Therapeutische Berichte 47 , 80-86, 1975). Hierzu wurde die vorinkubierte Probe mit 0,1 ml Kollagen, einem aggregationsauslösenden Agens, versetzt. Die Veränderung der optischen Dichte in der Probe der PRP wurde während einer Zeitdauer von 6 Minuten aufgezeichnet und der Ausschlag nach 6 Minuten bestimmt. Hierzu wird die prozentuale Hemmung gegenüber der Kontrolle errechnet.

Als Grenzkonzentration wird der Bereich der minimal effektiven Konzentration angegeben (Tabelle 1).

**Tabelle 1** **Thrombozytenaggregationshemmung**

| Beispiel-Nr. | Hemmung µg/ml (Grenzkonzentration) |
|---|---|
| 21 | 0,3-0,1 |
| 22 | 1,0-0,1 |
| 23 | 10 - 1 |
| 24 | 10 - 1 |
| 25 | 1,0 - 0,1 |
| 32 | 10 - 3 |
| 44 | 3,0 - 1,0 |

Als Maß für eine Lipoxygenase-Hemmung wurde die Freisetzung von Leukotrien $B_4$ ($LTB_4$) an polymorphkernigen Rattenleukozyten (PMN) nach Zugabe von Substanzen und Ca.-Ionophor mittels reverse phase HPLC nach Borgeat, P. et al, Proc. Nat. Acad. Sci. 76 , 2148-2152 (1979) bestimmt. Die in vivo-Aktivität der Verbindungen wurde mit dem Mäuseohr-Entzündungsmodell nach Young, J.M. et. al., J. of Investigative Dermatology 82 , 367-371 (1984) nachgewiesen.

In den Tabellen 2 und 3 sind beispielhaft die nach diesem Test erzielten Werte einiger erfindungsgemäße Verbindungen aufgeführt:

Tabelle 2 Lipoxygenasehemmung

| Bsp.-Nr. | $IC_{50}$-Wert (g/ml) |
|---|---|
| 27 | $8.8 \times 10^{-8}$ |
| 28 | $1.7 \times 10^{-7}$ |
| 31 | $3.3 \times 10^{-8}$ |
| 43 | $1.0 \times 10^{-7}$ |
| 44 | $1.0 \times 10^{-7}$ |
| 45 | $5.7 \times 10^{-8}$ |
| 46 | $4.6 \times 10^{-8}$ |
| 48 | $7.4 \times 10^{-8}$ |

Tabelle 3 Mouse Ear Inflammation Test

| Beispiel | Dosis | Entzündungshemmung % |
|---|---|---|
| 32 | 2 mg/Ohr top. | 58 |
| 45 | " | 39 |
| 48 | " | 65 |
| 23 | 100 mg/kg p.o. | 38 |
| 45 | " | 46 |
| 46 | " | 37 |

## Ansprüche

1. Phenylsulfonamide der Formel

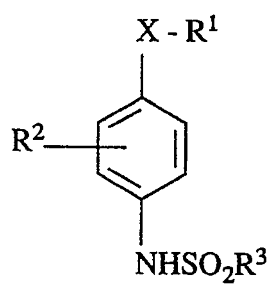

(I)

worin

$R^1$- für einen Chinolyl- oder Isochinolyrest steht, der substituiert sein kann durch Fluor, Chlor, Brom, $C_1$-$C_6$-Alkyl, Cyclopropyl, Cyclopentyl, Cyclohexyl, $C_1$-$C_6$-Alkoxy, Cyano, Trifluoromethyl Trifluomethoxy, Alkoxycarhonyl mit 1 bis 6 C-Atomen im Alkylteil, $C_1$-$C_6$-Alkylsulfonyl,

$R^2$- für Wasserstoff, Cyano, Nitro, Fluor, Chlor, Brom, $C_1$-$C_6$-Alkyl, $C_1$-$C_6$-Alkoxy Trifluoromethyl Trifluormethoxy oder Alkoxycarbonyl mit 1 bis C-Atomen im Alkylteil steht,

$R^3$- für Phenyl steht, das bis zu 2-fach gleich oder verschieden substituiert sein kann durch Fluor, Chlor, Brom, Trifluormethyl, Trifluormethoxy, $C_1$-$C_6$-AlKyl, $C_1$-$C_6$-Alkylthio, $C_1$-$C_6$-Alkylsulfonyl, Cyano, Alkoxycarbonyl mit 1 bis 6 C-Atomen im Alkylteil oder

- für Pentafluorphenyl oder
- für geradkettiges, verzweigtes oder cyclisches Alkyl mit bis zu 8 Kohlenstoffatomen steht, das substituiert sein kann durch Fluor, Chlor, Brom, Phenyl, Phenoxy, Cyano, $C_1$-$C_6$-Alkoxy oder Trifluormethyl und

X - für eine Gruppierung -O-, -A-B- oder -B-A- steht, wobei

A - -O -, 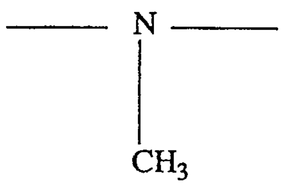 oder 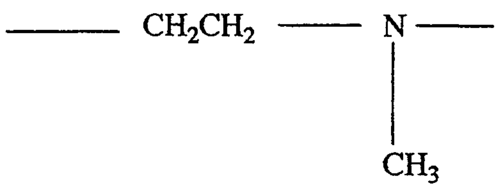 bedeutet

und

B - $-CH_2-$ oder 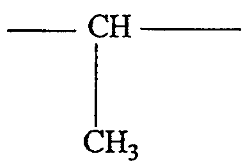 bedeutet,

und deren Salze.

2. Phenylsulfonamide der Formel 1 nach Anspruch 1 wobei

$R^1$- für einen Chinolyl- oder Isochinolylrest steht, der substituiert sein kann durch Fluor, Chlor, Alkyl mit bis zu 4 Kohlenstoffatomen, Alkoxy mit bis zu 4 Kohlenstoffatomen oder durch Trifluormethyl,

$R^2$- für Wasserstoff, Cyano, Fluor, Chlor, Methyl, Ethyl, Propyl, Isopropyl, Methoxy, Ethoxy, Trifluortmethyl, Methoxycarbonyl, Ethoxycarbonyl oder Propoxycarbonyl steht,

$R^3$- für Phenyl steht, das substituiert sein kann durch Fluor, Chlor, Trifluormethyl, Trifluoromethoxy Alkyl mit bis zu 4 Kohlenstoffatomen oder

- für Pentafluorphenyl oder
- für geradkettiges oder verzweigtes Alkyl mit bis zu 6 Kohlenstoffatomen steht, das substituiert sein kann durch Fluor, Chlor oder Phenyl und

X -. für -O-, $-OCH_2$, $-CH_2O-$, $-OCH(CH_3)-$, $CH_2N(CH_3)-$, $-CH_2N(CH_3)CH_2CH_2-$ steht,

und deren Salze.

3. Phenylsulfonamide nach den Ansprüchen 1 und 2 zur therapeutischen Behandlung.

4. Verfahren zur Herstellung von Phenylsulfonamiden der Formel (I)

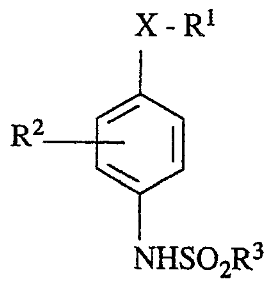

(I)

worin

$R^1$- für einen Chinolyl- oder Isochinolylrest steht, der substituiert sein kann durch Fluor, Chlor, Brom, $C_1$-$C_6$-Alkyl, Cyclopropyl, Cyclohexyl, Cyclohexyl, $C_1$-$C_6$-Alkoxy, Cyano,Trifluoromethyl, Trifluorrnethoxy, Alkoxycarbonyl mit 1 bis 6C-Atomen im Alkylteil, $C_1$-$C_6$-Alkylsulfonyl,

$R^2$- für Wasserstoff, Cyano, Nitro, Fluor, Chlor, Brom, $C_1$-$C_6$-Alkyl, $C_1$-$C_6$-Alkoxy, Trifluoromethyl, Trifluormethoxy oder Alkoxycarbonyl mit 1 bis 6 C-Atomen im Alkylteil steht,

$R^3$- für Phenyl steht, das bis zu 2-fach gleich oder verschieden substituiert sein kann durch Fluor, Chlor, Brom, Trifluormethyl, Trifluormethoxy, $C_1$-$C_6$-Alkyl, $C_1$-$C_6$-Alkylthio, $C_1$-$C_6$-Alkylsulfonyl, Cyano, Alkoxycarbonyl mit 1 bis 6 C-Atomen im Alkylteil oder

- für Pentafluorphenyl oder
- für geradkettiges, verzweigtes oder cyclisches Alkyl mit bis zu 8 Kohlenstoffatomen steht, das substituiert sein kann durch Fluor, Chlor, Brom, Phenyl, Phenoxy, Cyano, $C_1$-$C_6$-Alkoxy oder Trifluormethyl

und

X - für eine Gruppierung -O-, -A-B- oder -B-A- steht,
wobei

A - -O -, 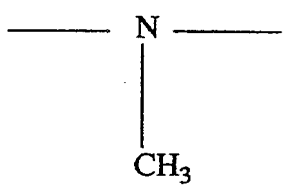 oder 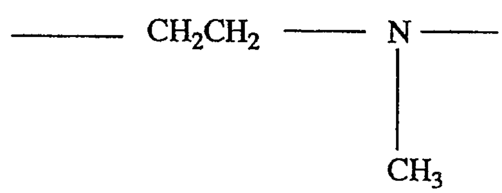 bedeutet

und

B - $-CH_2-$ oder 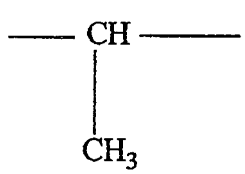 bedeutet,

und deren Salze,
dadurch gekennzeichnet, daß man Amine der allgemeinen Formel II

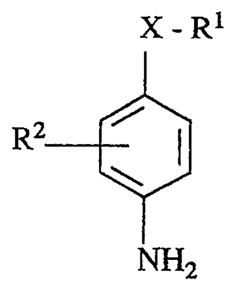

(II)

in welcher
$R^1$, $R^2$ und X die oben angegebene Bedeutung haben, mit Sulfonsäurehalogeniden der allgemeinen Formel (III)

$$R^3\text{-}SO_2\text{-}Y \quad (III)$$

in welcher
$R^3$- die oben angegebene Bedeutung hat,
und
Y - für Halogen steht,
in Gegenwart eines inerten Lösemittels, gegebenenfalls in Gegenwart einer Base umsetzt und dann gegebenenfalls im Fall der Herstellung der Salze mit einer entsprechenden Säure umsetzt.

**5.** Verfahren nach Anspruch 4, dadurch gekennzeichnet, daß man die Umsetzung im Temperaturbereich von -30° C bis +150° C durchführt.

**6.** Arzneimittel enthaltend ein oder mehrere Phenylsulfonamide nach Anspruch 1.

**7.** Arzneimittel nach Anspruch 6 enthaltend 0,5 bis 90 Gew.-% Phenylsulfonamide, bezogen auf die Gesamtmischung.

**8.** Verwendung von Phenylsulfonamiden nach Anspruch 1 zur Herstellung von Arzneimitteln.

**9.** Verwendung nach Anspruch 8 zur Herstellung von Lipoxygenasehemmern.

**10.** Verwendung nach Anspruch 8 zur Herstellung von Thrombozytenaggregationshemmern.

Patentansprüche für folgenden Vertragsstaat : ES

**1.** Verfahren zur Herstellung von Phenylsulfonamiden der Formel (I)

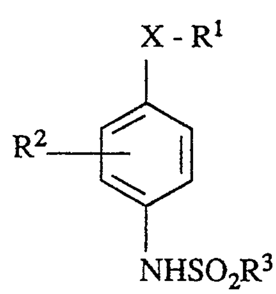

(I)

worin
$R^1$- für einen Chinolyl- oder Isochinolylrest steht, der substituiert sein kann durch Fluor, Chlor, Brom, $C_1$-$C_6$-Alkyl, Cyclopropyl, Cyclopentyl, Cyclohexyl, $C_1$-$C_6$-Alkoxy, Cyano, Trifluormethyl, Trifluormethoxy, Alkoxycarbonyl mit 1 bis 6 C-Atomen im Alkylteil, $C_1$-$C_6$-Alkylsulfonyl,

$R^2$- für Wasserstoff, Cyano, Nitro, Fluor, Chlor, Brom, $C_1$-$C_6$-Alkyl, $C_1$-$C_6$-Alkoxy, Trifluormethyl, Trifluormethoxy oder Alkoxycarbonyl mit 1 bis 6 C-Atomen im Alkylteil steht,

$R^3$- für Phenyl steht, das bis zu 2-fach gleich oder verschieden substituiert sein kann durch Fluor, Chlor, Brom, Trifluormethyl, Trifluormethoxy, $C_1$-$C_6$-Alkyl, $C_1$-$C_6$-Alkylthio, $C_1$-$C_6$-Alkylsulfonyl, Cyano, Alkoxycarbonyl mit 1 bis 6 C-Atomen im Alkylteil oder

- für Pentafluorphenyl oder
- für geradkettiges, verzweigtes oder cyclisches Alkyl mit bis zu 8 Kohlenstoffatomen steht, das substituiert sein kann durch Fluor, Chlor, Brom, Phenyl, Phenoxy, Cyano, $C_1$-$C_6$-Alkoxy oder Trifluormethyl und

x - für eine Gruppierung -O-, -A-B- oder -B-A- steht, wobei

A - -O-, 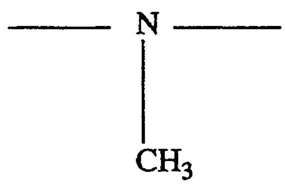 oder 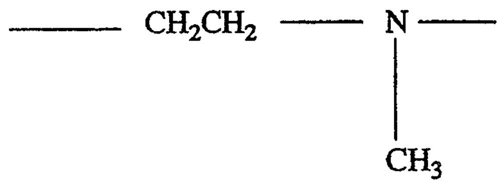 bedeutet

und

B - $-CH_2-$ oder 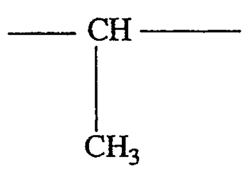 bedeutet,

und deren Salze,
dadurch gekennzeichnet, daß man Amine der allgemeinen Formel II

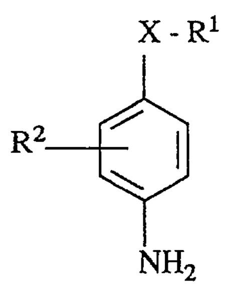 (II)

in welcher
$R^1$, $R^2$ und X die oben angegebene Bedeutung haben, mit Sulfonsäurehalogeniden der allgemeinen Formel (III)

$$R^3\text{-}SO_2\text{-}Y \quad (III)$$

in welcher
$R^3$- die oben angegebene Bedeutung hat, und
Y - für Halogen steht,
in Gegenwart eines inerten Lösemittels, gegebenenfalls in Gegenwart einer Base umsetzt und dann gegebenenfalls im Fall der Herstellung der Salze mit einer entsprechenden Säure umsetzt.

2. Verfahren nach Anspruch 1 zur Herstellung von Phenylsulfonamiden der Formel nach Anspruch 1, worin
   - $R^1$ - für einen Chinolyl- oder Isochinolylrest steht, der substituiert sein kann durch Fluor, Chlor, Alkyl mit bis zu 4 Kohlenstoffatomen, Alkoxy mit bis zu 4 Kohlenstoffatomen oder durch Trifluormethyl,
   - $R^2$ - für Wasserstoff, Cyano, Fluor, Chlor, Methyl, Ethyl, Propyl, Isopropyl, Methoxy, Ethoxy, Trifluormethyl, Methoxycarbonyl, Ethoxycarbonyl oder Propoxycarbonyl steht,
   - $R^3$ - für Phenyl steht, das substituiert sein kann durch Fluor, Chlor, Trifluormethyl, Trifluormethoxy, Alkyl mit bis zu 4 Kohlenstoffatomen
     oder
     - für Pentafluorphenyl oder
     - für geradkettiges oder verzweigtes Alkyl mit bis zu 6 Kohlenstoffatomen steht, das substituiert sein kann durch Fluor, Chlor oder Phenyl und
   - X - für -O-, $-OCH_2-$, $-CH_2O-$, $-OCH(CH_3)-$, $CH_2N(CH_3)-$, $-CH_2N(CH_3)CH_2CH_2-$ steht
     und deren Salze.

3. Verfahren nach Anspruch 1 , dadurch gekennzeichnet, daß man die Umsetzung im Temperaturbereich von -30° C bis +150° C durchführt.

4. Verfahren nach Anspruch 1,dadurch gekennzeichnet daß man die Umsetzung in einem Temperaturbereich von -20° C bis +80° C durchführt.

5. Verfahren nach Anspruch 1 , dadurch gekennzeichnet, daß man 1 bis 5 Mol Sulfonsäurehalogenid, bezogen auf 1 Mol des Amins einsetzt.

6. Verfahren nach Anspruch 1, dadurch gekennzeichnet, daß man als Basen Alkali- oder Erdalkalihydroxyde, Alkalihydride, Alkali- oder Erdalkalicarbonate, Alkalialkoholate, Alkaliamide oder organische Amine einsetzt.

## Claims

1. Phenylsulphonamides of the formula (I)

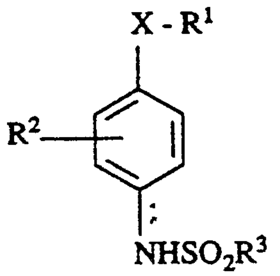

(I)

in which
- $R^1$ represents a quinolyl or isoquinolyl radical which may be substituted by fluorine, chlorine, bromine, $C_1$-$C_6$-alkyl, cyclopropyl, cyclopentyl, cyclohexyl, $c_1$-$C_6$-alkoxy, cyano, trifluoromethyl, trifluoromethoxy, alkoxycarbonyl having 1 to 6 C atoms in the alkyl moiety or $C_1$-$C_6$-alkylsulphonyl,
- $R^2$ represents hydrogen, cyano, nitro, fluorine, chlorine, bromine, $C_1$-$C_6$-alkyl, $C_1$-$C_6$-alkoxy, trifluoromethyl, trifluoromethoxy or alkoxycarbonyl having 1 to 6 C atoms in the alkyl moiety,

R[3] represents phenyl which may be monosubstituted or disubstituted by fluorine, chlorine, bromine, trifluoromethyl, trifluoromethory, $C_1$-$C_6$-alkyl, $C_1$-$C_6$-alkyl-thio, $C_1$-$C_6$-alkylsulphonyl, cyano or alkoxycarbonyl having 1 to 6 C atoms in the alkyl moiety, the substituents being identical or different, or
represents pentafluorophenyl, or
represents straight-chain, branched or cyclic alkyl, having up to 8 carbon atoms, which may be substituted by fluorine, chlorine, bromine, phenyl, phenoxy, cyano, $C_1$-$C_6$-alkoxy or trifluoromethyl,
and

x represents an -0-, -A-B- or -B-A- group, where

A denotes -O-, 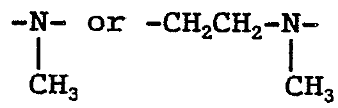

and

B denotes 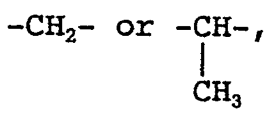

and the salts thereof.

2. Phenylsulphonamides of the formula (I) according to Claim 1, where
   R[1] represents a quinolyl or isoquinolyl radical which may be substituted by fluorine, chlorine, alkyl having up to 4 carbon atoms, alkory having up to 4 carbon atoms or by trifluoromethyl,
   R[2] represents hydrogen, cyano, fluorine, chlorine, methyl, ethyl, propyl, isopropyl, methoxy, ethory, trifluoromethyl, methoxycarbonyl, ethoxycarbonyl or propoxycarbonyl,
   R[3] represents phenyl which may be substituted by fluorine, chlorine, trifluoromethyl, trifluoromethoxy, alkyl having up to 4 carbon atoms or represents pentafluorophenyl, or represents straight-chain or branched alkyl, having up to 6 carbon atoms, which may be substituted by fluorine, chlorine or phenyl,
   and
   x represents -0-, $-0CH_2-$, $-CH_20-$, $-0CH(CH_3)-$, $-CH_2N(CH_3)$ - or $-CH_2N(CH_3)CH_2CH_2-$, and the salts thereof.

3. Phenylsulphonamides according to Claims 1 and 2, for therapeutic treatment.

4. Process for the preparation of phenylsulphonamides of the formula (I)

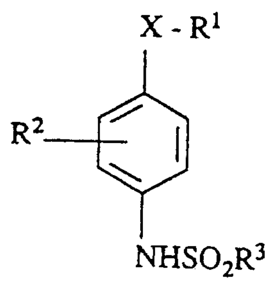

(I)

in which
   R[1] represents a guinolyl or isoguinolyl radical which may be substituted by fluorine, chlorine, bromine, $C_1$-$C_6$-alkyl, cyclopropyl, cyclopentyl, cyclohexyl, $C_1$-$C_6$-alkoxy, cyano, trifluoromethyl, trifluoromethoxy, alkoxycarbonyl having 1 to 6 C atoms in the alkyl moiety or $C_1$-$C_6$-alkylsulphonyl,
   R[2] represents hydrogen, cyano, nitro, fluorine, chlorine, bromine, $C_1$-$C_6$-alkyl, $C_1$-$C_6$-alkoxy, trifluoromethyl, trifluoromethoxy or alkoxycarbonyl having 1 to 6 C atoms in the alkyl moiety,

$R^3$ represents phenyl which may be mono-substituted or disubstituted by fluorine, chlorine, bromine, trifluoromethyl, trifluoromethoxy, $C_1$-$C_6$-alkyl, $C_1$-$C_6$-alkyl-thio, $C_1$-$C_6$-alkylsulphonyl, cyano or alkoxycarbonyl having 1 to 6 C atoms in the alkyl moiety, the substituents being identical or different, or
represents pentafluorophenyl, or
represents straight-chain, branched or cyclic alkyl, having up to 8 carbon atoms, which may be substituted by fluorine, chlorine, bromine, phenyl, phenoxy, cyano, $C_1$-$C_6$-alkoxy or trifluoromethyl,
and

x represents an -0-, -A-B- or -B-A- group, where

A denotes -O-, 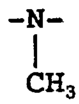 or 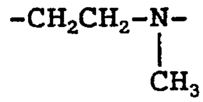

and

B denotes $-CH_2-$ or 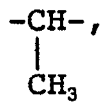,

characterized in that
amines of the general formula (II)

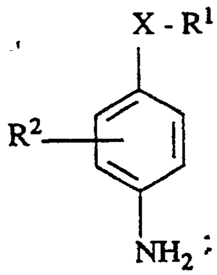

(II)

in which
$R^1$, $R^2$ and X have the abovementioned meaning, are reacted with sulphonyl halides of the general formula (III)

$$R^3\text{-}SO_2\text{-}Y \quad (III)$$

in which
$R^3$ has the abovementioned meaning
and
Y represents halogen
in the presence of an inert solvent and if appropriate in the presence of a base, and, in the case of the preparation of the salts, the product of this reaction is reacted, if appropriate, with an appropriate acid.

5. Process according to Claim 4, characterized in that the reaction is carried out in the temperature range from -30 ° C to +150 ° C.

6. Medicament containing one or more phenylsulphonamides according to Claim 1.

7. Medicaments according to Claim 6, containing 0.5 to 90% by weight of phenylsulphonamides, relative to the total mixture.

8. Use of phenylsulphonamides according to Claim 1 for the preparation of medicaments.

9. Use according to Claim 8 for the preparation of lipoxygenase inhibitors.

10. Use according to Claim 8 for the preparation of thrombocyte-aggregation inhibitors.

Claims for the following Contracting State : ES

1. Process for the preparation of phenylsulphonamides of the formula (I)

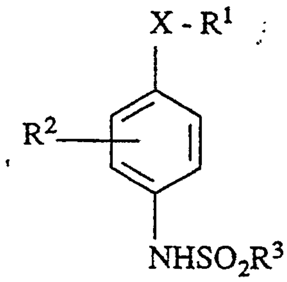

(I)

in which

$R^1$ represents a guinolyl or isoguinolyl radical which may be substituted by fluorine, chlorine, bromine, $C_1$-$C_6$-alkyl, cyclopropyl, cyclopentyl, cyclohexyl, $C_1$-$C_6$-alkoxy, cyano, trifluoromethyl, trifluoromethoxy, alkoxycarbonyl having 1 to 6 C atoms in the alkyl moiety or $C_1$-$C_6$-alkylsulphonyl,

$R^2$ represents hydrogen, cyano, nitro, fluorine, chlorine, bromine, $C_1$-$C_6$-alkyl, $C_1C_6$-alkoxy, trifluoromethyl, trifluoromethoxy or alkoxycarbonyl having 1 to 6 C atoms in the alkyl moiety,

$R^3$ represents phenyl which may be monosubstituted or disubstituted by fluorine, chlorine, bromine, trifluoromethyl, trifluoromethoxy, $C_1$-$C_6$-alkyl, $C_1$-$C_6$-alkyl-thio, $C_1$-$C_6$-alkylsulphonyl, cyano or alkoxycarbonyl having 1 to 6 C atoms in the alkyl moiety, the substituents being identical or different, or
represents pentafluorophenyl, or
represents straight-chain, branched or cyclic alkyl, having up to 8 carbon atoms, which may be substituted by fluorine, chlorine, bromine, phenyl, phenoxy, cyano, $C_1$-$C_6$-alkoxy or trifluoromethyl,
and

X represents an -0-, -A-B- or -B-A- group, where

A denotes 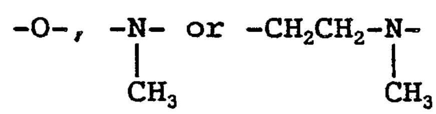

and

B denotes 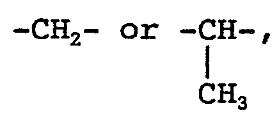

and the salts thereof, characterized in that amines of the general formula (II)

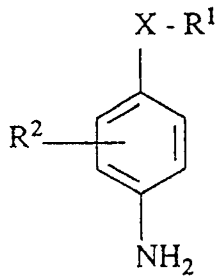

(II)

in which

$R^1$, $R^2$ and X have the abovementioned meaning, are reacted with sulphonyl halides of the general formula (III)

$$R^3\text{-}SO_2\text{-}Y \quad (III)$$

in which

$R^3$ has the abovementioned meaning and

Y represents halogen

in the presence of an inert solvent and if appropriate in the presence of a base, and, in the case of the preparation of the salts, the product of this reaction is reacted, if appropriate, with an appropriate acid.

**2.** Process according to Claim 1 for the preparation of phenylsulphonamides of the formula according to Claim 1, in which

$R^1$ represents a guinolyl or isoguinolyl radical which may be substituted by fluorine, chlorine, alkyl having up to 4 carbon atoms, alkoxy having up to 4 carbon atoms or by trifluoromethyl,

$R^2$ represents hydrogen, cyano, fluorine, chlorine, methyl, ethyl, propyl, isopropyl, methoxy, ethoxy, trifluoromethyl, methoxycarbonyl, ethoxycarbonyl or propoxycarbonyl,

$R^3$ represents phenyl which may be substituted by fluorine, chlorine, trifluoromethyl, trifluoromethoxy, alkyl having up to 4 carbon atoms or represents pentafluorophenyl, or represents straight-chain or branched alkyl, having up to 6 carbon atoms, which may be substituted by fluorine, chlorine or phenyl, and

X represents -0-, $-0CH_2-$, $-CH_20-$,$-0CH(CH_3)-$, $-CH_2N(CH_3)$ - or $-CH_2N(CH_3)CH_2CH_2-$, and the salts thereof.

**3.** Process according to Claim 1, characterized in that the reaction is carried out in the temperature range from -30 °C to +150 °C.

**4.** Process according to Claim 1, characterized in that the reaction is carried out in a temperature range from -20 °C to +8O °C.

**5.** Process according to Claim 1, characterized in that 1 to 5 mol of sulphonyl, halide, based on 1 mol of the amine, is used.

**6.** Process according to Claim 1, characterized in that the bases used are alkali metal or alkaline earth metal hydroxides, alkali metal hydrides, alkali metal or alkaline earth metal carbonates, alkali metal alkoxides, alkali metal amides or organic amines.

## Revendications

**1.** Phénylsulfonamides de la formule

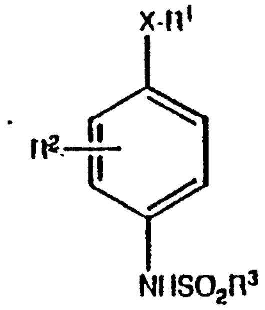

(I)

dans laquelle

$R^1$- représente un résidu quinolyle ou isoquinolyle qui peut être substitué par un fluor, un chlore, un brome, un alkyle $C_1$-$C_6$, un cycopropyle, un cyclopentyle, un cyclohexyle, un alkoxy $C_1$-$C_6$, un cyano, un trifluorométhyle, un trifluorométhoxy, un alkoxycarbonyle avec 1 à 6 atomes C dans la partie alkyle, un alkylsulfonyle $C_1$-$C_6$;

$R^2$- représente un hydrogène, un cyano, un nitro, un fluor, un chlore, un brome, un alkyle $C_1$-$C_6$, un alkoxy $C_1$-$C_6$, un trifluorométhyle, un trifluorométhoxy ou un alkoxy-carbonyle avec 1 à 6 atomes C dans la partie alkyle,

$R^3$- représente un phényle qui peut être substitué deux fois, identiques ou différentes, par un fluor, un chlore, un brome, un trifluorométhyle, un trifluorométhoxy, un alkyle $C_1$-$C_6$, un alkylthio $C_1$-$C_6$, un alkylsulfonyle $C_1$-$C_6$, un cyano, un alkoxycarbonyle avec 1 à 6 atomes C dans la partie alkyle ou

- un pentafluorophényle ou
- un alkyle à chaine droite, ramifiée ou cyclique avec 8 atomes de carbone maximum qui peut être substitué par un fluor, un chlore, un brome, un phényle, un phénoxy, un cyano, un alkoxy $C_1$-$C_6$ ou un trifluorométhyle

et

X- représente un groupe -0-, -A-B- ou -B-A,
où

**-A- désigne - 0 -,** 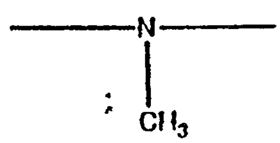 ou 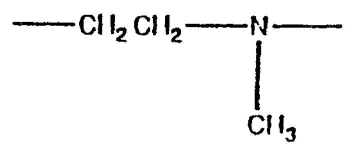

**et**

**-B- désigne** $—CH_2—$ ou 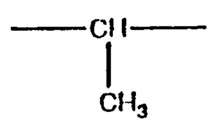

et leurs sels.

**2.** Phénylsulfonamides de la formule I selon la revendication 1
où

$R^1$- représente un résidu quinolyle ou isoquinolyle qui peut être substitué par un fluor, un chlore, un alkyle avec au maximum 4 atomes de carbone, un alkoxy avec 4 atomes de carbone maximum et un trifluorométhyle;

$R^2$- représente un hydrogène, un cyano, un fluor, un chlore, un méthyle, un éthyl, un propyl, un isopropyl, un méthoxy, un éthoxy, un trifluorométhyle, un méthoxycarbonyle, un éthoxycarbonyle ou un propoxycarbonyle;

$R^3$- représente un phényle qui peut être substitué par un fluor, un chlore, un trifluorométhyle, un trifluorométhoxy, un alkyle avec maximum 4 atomes de carbone
ou

- un pentafluorophényl ou
- un alkyle ramifié ou linéaire avec 6 atomes de carbone maximum qui peut être substitué par un fluor, un chlore, ou un phényle et

X- représente -0-, $-OCH_2-$, $-CH_2O-$, $-OCH(CH_3)-$, $CH_2N\ (CN_3)-$, $-CH_2\ N\ (CH_3)\ CH_2CH_2-$ et leurs sels.

**3.** Phénylsulfonamides selon les revendications 1 et 2 pour le traitement thérapeutique.

**4.** Procédé de fabrication des phénylsulfonamides de la formule (I)

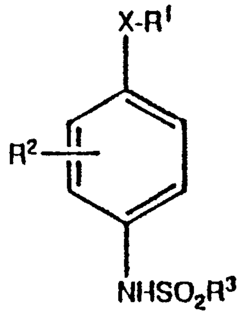

(I)

dans laquelle

$R^1$- représente un résidu quinolyle ou isoquinolyle qui peut être substitué par un fluor, un chlore, un brome, un alkyle $C_1$ -$C_6$, un cyclopropyle, un cyclopentyle, un cyclohexyle, un alkoxy $C_1$-$C_6$, un cyano, un trifluorométhyle, un trifluorométhoxy, un alkoxycarbonyle avec 1 à 6 atomes C dans la partie alkyle, un alkylsulfonyle $C_1$-$C_6$;

$R^2$- représente un hydrogène, un cyano, un nitro, un fluor, un chlore, un brome, un alkyle $C_1$-$C_5$, un alkoxy $C_1$-$C_6$, un trifluorométhyle, un trifluorométhoxy ou un alkoxycarbonyle avec 1 à 6 atomes C dans la partie alkyle,

$R^3$- représente un phényle qui peut être substitué deux fois, identiques ou différentes, par un fluor, un chlore, un brome, un trifluorométhyle, un trifluorméthoxy, un alkyle $C_1$-$C_6$, un alkylthio $C_1$-$C_6$, un alkylsulfonyle $C_1$-$C_6$, un cyano, un alkoxycarbonyle avec 1 à 6 atomes C dans la partie alkyle ou

- un pentafluorophényle ou
- un alkyle à chaîne droite, ramifiée ou cyclique avec 8 atomes de carbone maximum qui peut être substitué par un fluor, un chlore, un brome, un phényle, un phénoxy, un cyano, un alkoxy $C_1$-$C_6$ ou un trifluorométhyle

et

X- représente un groupe -O-, -A-B- ou -B-A,
Où

**-A- désigne -O-** 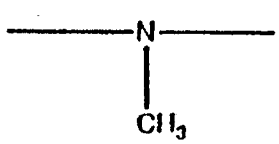 ou 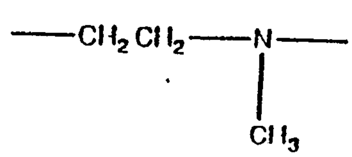

**et**

**-B-** **désigne** $-CH_2-$ ou 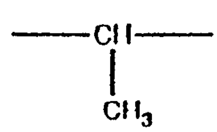

et leurs sels,
caractérisé en ce qu'une amine de la formule générale II

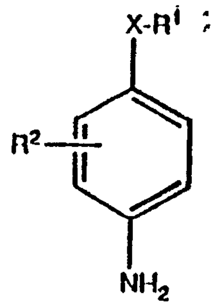

(II)

dans laquelle
$R^1$, $R^2$ et X ont la signification susmentionnée est mise en réaction avec un halogénure sulfonique de la formule générale (III)

$$R^3—SO_2—Y \qquad (III)$$

dans laquelle
$R^3$ a la signification susmentionnée,
et
Y- représente un halogène,
en présence d'un solvant inerte, le cas échéant en présence d'une base et puis, éventuellement, avec un acide correspondant, en cas de fabrication de sels.

**5.** Procédé selon la revendication 4, caractérisé en ce que la réaction se déroule à une température de -30 °C à +150 °C.

**6.** Médicaments contenant un ou plusieurs sulfonamides selon la revendication 1.

**7.** Médicaments selon la revendication 6 contenant 0,5 à 90 % en poids de phénylsulfonamide par rapport au mélange total.

**8.** Utilisation de phénylsulfonamides selon la revendication 1 pour la fabrication de médicaments.

**9.** Utilisation selon la revendication 8 pour la fabrication d'inhibiteurs de la lipoxygénase.

**10.** Utilisation selon la revendication 8 pour la fabrication d'inhibiteurs de l'agrégation thrombocytaire.

Revendications pour l'Etat contractant suivant : ES

**1.** Procédé de fabrication de phénylsulfonamides de la formule (I)

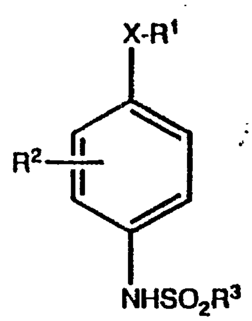

(I)

dans laquelle

$R^1$- représente un groupe quinolyle ou isoquinolyle qui peut être substitué par un fluor, un chlore, un brome, un alkyle $C_1$-$C_8$, un cyclopropyle, un cyclopentyle, un cyclohexyle, un alkoxy $C_1$-$C_6$, un cyano, un trifluorométhyle, un trifluorométhoxy, un alkoxycarbonyle avec 1 à 6 atomes C dans la partie alkyle, un alkylsulfonyle $C_1$-$C_6$;

$R^2$- représente un hydrogène, un cyano, un nitro, un fluor, un chlore, un brome, un alkyle $C_1$-$C_6$, un alkoxy $C_1$-$C_6$, un trifluorométhyle, un trifluorométhoxy ou un alkoxy-carbonyle avec 1 à 6 atomes C dans la partie alkyle,

$R^3$- représente un phényle qui peut être substitué deux fois, identiques ou différentes, par un fluor, un chlore, un brome, un trifluorométhyle, un trifluorométhoxy, un alkyle $C_1$-$C_6$, un alkylthio $C_1$-$C_6$, un alkylsulfonyle $C_1$-$C_6$, un cyano, un alkoxycarbonyle avec 1 à 6 atomes C dans la partie alkyle ou

- un pentafluorophényle ou
- un alkyle à chaîne droite, ramifiée ou cyclique avec 8 atomes de carbone maximum qui peut être substitué par un fluor, un chlore, un brome, un phényle, un phénoxy, un cyano, un alkoxy $C_1$-$C_6$ ou un trifluorométhyle

et

X- représente un groupe -O-, -A-B- ou -B-A, où

**-A- désigne - 0 -,** 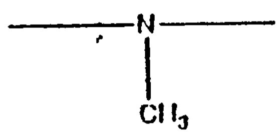 ou 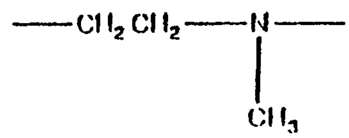

**et**

**-B- désigne** $-CH_2-$ ou 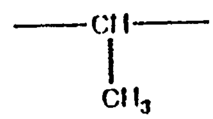

et leurs sels.
caractérisé en ce qu'
un amine de la formule générale II

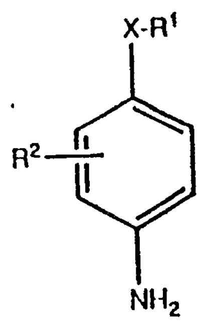 (II)

dans laquelle
R1, R2 et X ont la signification susmentionnée est mise en réaction avec un halogénure sulfonique de la formule générale (III)

$$R^3-SO_2-Y \quad (III)$$

dans laquelle
R3- a la signification susmentionnée
et
Y - représente un halogène

en présence d'un solvant inerte, le cas échéant en présence d'une base et puis, le cas échéant, avec un acide correspondant en cas de fabrication de sels.

**2.** Procédé selon la revendication 1 de fabrication de phénylsulfamides de la formule selon la revendication 1,
où
$R^1$ - représente un résidu quinolyle ou isoquinolyle qui peut être substitué par un fluor, un chlore, un alkyle avec au maximum 4 atomes de carbone, un alkoxy avec 4 atomes de carbone maximum et un trifluorométhyle;
$R^2$- représente un hydrogène, un cyano, un fluor, un chlore, un méthyle, un éthyle, un propyle, un isopropyle, un méthoxy, un éthoxy, un trifluorométhyle, un méthoxycarbonyle, un éthoxycarbonyle ou un propoxycarbonyle;
$R^3$- représente un phényle qui peut être substitué par un fluor, un chlore, un trifluorométhyle, un trifluorométhoxy, un alkyle avec maximum 4 atomes de carbone
ou
- un pentafluorophényle ou
- un alkyle à chaîne linéaire ou ramifiée avec 6 atomes de carbone maximum qui peut être substitué par un fluor, un chlore, ou un phényle et

X- représente -O-, $-OCH_2-$, $-CH_2O-$, $-OCH(CH_3)-$, $CH_2N(CH_3)-$, $CH_2N(CH_3)CH_2CH_2-$ et leurs sels.

**3.** Procédé selon la revendication 1, caractérisé en ce que la réaction se déroule à une température de -30 °C à +150 °C.

**4.** Procédé selon la revendication 1 caractérisé en ce que la réaction se déroule à une température de -20 °C à +80 °C.

**5.** Procédé selon la revendication 1 caractérisé en ce qu'on utilise 1 à 5 moles d'halogénure sulfonique pour 1 mole d'amine.

**6.** Procédé selon la revendication 1 caractérisé en ce que les bases utilisées sont des hydroxydes alcalins ou alcalino-terreux, des hydrures alcalins, des carbonates alcalins ou alcalino-terreux, des alcoolates alcalins, des amides alcalins ou des amines organiques.